# EUROPÄISCHE PATENTSCHRIFT

(11) **EP 0 886 679 B1**
(45) Veröffentlichungstag und Bekanntmachung des Hinweises auf die Patenterteilung: **06.06.2007**
(21) Anmeldenummer: 97904453.4
(22) Anmeldetag: 17.02.1997
(51) Int. Cl.: C12N 15/86, C12N 15/39, C07K 14/065, C12N 7/01, A61K 39/275, C12N 15/11

(54) **PARAPOCKENVIREN, DIE FREMD-DNA ENTHALTEN, IHRE HERSTELLUNG UND IHRE VERWENDUNG IN IMPFSTOFFEN**
PARAPOXVIRUSES CONTAINING FOREIGN DNA, THEIR PRODUCTION AND THEIR USE IN VACCINES
PARAPOXVIRUS CONTENANT DE L'ADN ETRANGER, LEUR PRODUCTION ET LEUR UTILISATION DANS DES VACCINS

(30) Priorität: 28.02.1996 DE 19607458; 26.09.1996 DE 19639601
(43) Veröffentlichungstag der Anmeldung: 30.12.1998
(73) Patentinhaber: Bayer HealthCare AG, 51368 Leverkusen (DE)
(72) Erfinder: SCHMEER, Norbert, D-42781 Haan (DE); STRUBE, Walter, D-50259 Pulheim (DE); BÜTTNER, Mathias, D-72076 Tübingen (DE); RZIHA, Hans-Joachim, D-50858 Köln (DE)
(86) Internationale Anmeldenummer: PCT/EP1997/000729
(87) Internationale Veröffentlichungsnummer: WO 1997/032029

(56) Entgegenhaltungen:
- DE-A- 4 405 841
- JOURNAL OF GENERAL VIROLOGY, Bd. 76, Nr. 12, Dezember 1995, READING GB, Seiten 2969-2978, XP002032167 S.B. FLEMING ET AL.: "Genomic analysis of a transposition-deletion variant of orf virus reveals a 3.3kbp region of non-essential DNA"
- JOURNAL OF VIROLOGY, Bd. 68, Nr. 1, Januar 1994, Seiten 84-92, XP002032168 D.J. LYTTLE: "Homologs of vascular endothelial growth factor are encoded by the poxvirus Orf virus"
- VIROLOGY, Bd. 212, Nr. 2, 1.Oktober 1995, ORLANDO US, Seiten 698-704, XP002032169 A.A. MERCER ET AL.: "The establishment of a genetic map of Orf virus reveals a pattern of genomic organization that is highly conserved among divergent poxviruses"

## Beschreibung

Gegenstand der vorliegenden Erfindung sind rekombinante Parapockenviren, ihre Herstellung, Impfstoffe und Immunmodulatoren, die sie enthalten.

Die erfindungsgemäßen gentechnisch veränderten Parapockenviren tragen in ihrem Genom Deletionen und/oder Insertionen. Die Deletion von Genomabschnitten der Parapockenviren und/oder die Insertion von Fremd-DNA kann zur Reduktion bzw. zum Verlust ihrer Pathogenität (Attenuierung) führen. Durch Insertionen werden Erbinformationen von Erregern oder biologisch wirksamen Substanzen in das Genom der Parapockenviren eingebaut. Diese fremden Erbinformationen werden als Bestandteil der rekombinanten Parapochenviren, beispielsweise in Zellkulturen, Geweben oder in intakten Organismen, zur Expression gebracht.

Die erfindungsgemäß hergestellten rekombinanten Parapockenviren werden z.B. in Vakzinen oder Immunmodulatoren eingesetzt. Die Expression der Fremd-DNA im Genom der Parapockenviren löst z.B. im Impfling eine Abwehrreaktion gegen die Erreger aus, die durch die fremde Erbinformation repräsentiert werden. Zusätzlich können die nicht spezifischen Abwehrkräfte des Impflings stimuliert werden. (Im folgenden wird der Begriff Parapockenviren durch PPV abgekürzt.)

PPV selbst können immunmodulatorisch wirken, da sie nicht-erregerspezifische Immunreaktionen im Organismus stimulieren. So werden Präparationen aus Parapockenviren beispielsweise in der Tiermedizin zur Steigerung der allgemeinen Abwehrkräfte erfolgreich eingesetzt

Vakzinen, die erregerspezifisch wirken, benötigen in Abhängigkeit des Antigens für die Etablierung des Schutzes mehrere Tage bis Wochen, bieten aber dann einen langen, über Monate bis Jahre anhaltenden Schutz.

Vakzinen, die auf Basis von rekombinanten Parapockenviren hergestellt werden, können somit als biologische Produkte für eine verbesserte Bekämpfung von Infektionskrankheiten eingesetzt werden, da sie sowohl eine langanhaltende, erregerspezifische Immunität aufbauen als auch einen sehr schnell eintretenden erregerunspezifischen Schutz im Organismus induzieren.

Die Kombination der immunstimulierenden Eigenschaften der PPV mit der Expression von Fremdantigenen, die einen homologen und/oder heterologen erregerspezifischen Schutz induzieren ist neu. Dies erlaubt die Herstellung von Produkten, die sowohl einen schnell einsetzenden, breiten erregerunspezifischen Schutz gegen Infektionen vermitteln als auch einen langanhaltenden, erregerspezifischen Infektionsschutz bieten.

Die Familie der Vertebraten-Pockenviren (Chordopoxvirinae) wird in einzelne, eigenständige Gattungen (Genera) unterteilt. Die vorliegende Erfindung betrifft die Gattung der PPV, die sich sowohl strukturell als auch genetisch von den übrigen Pockenviren unterscheiden. Die PPV werden in drei verschiedene Spezies eingeteilt (Lit. #1):
- Parapoxvirus ovis (auch Ecthyma Contagiosum Virus, Virus der Kontagiösen Pustulardermitis oder Orf Virus genannt), das als Prototyp des Genus gilt,
- Parapoxvirus bovis 1 ( auch Bovine Papulöse Stomatitis Virus oder Stomatitis Papulosa Virus genannt) und
- Parapoxvirus bovis 2 (auch Euterpocken Virus, Paravaccinia Virus, Pseudokuhpocken Virus oder Melkerknoten Virus genannt).

Es sind auch Parapockenvirus-Vertreter beschrieben, die aus Kamelen, Rothirschen, Gemsen, Robben, Seehunden und Seelöwen isoliert wurden. Ob es sich hierbei um eigenständige Spezies innerhalb der Gattung Parapockenviren oder um Isolate der oben beschriebenen Spezies handelt, ist noch nicht endgültig geklärt.

Infektionen mit PPV können lokale Erkrankungen beim Tier und beim Menschen (Zoonose-Erreger) hervorrufen. Lit. #1 gibt einen Überblick über die bislang beschriebenen Krankheitsbilder. Die Bekämpfung der Erkrankungen ist möglich durch den Einsatz von prophylaktischen Maßnahmen, wie beispielsweise Vakzinen. Die bislang erhältlichen Vakzinen, die ausschließlich auf Basis von Parapopxvirus ovis entwickelt wurden, zeigen allerdings unbefriedigende Wirksamkeit (Lit. #2).

Gegenstand der Erfindung ist es, das PPV als Träger (Vektor) von fremder genetischer Information, die exprimiert wird, zu nutzen.

Vektoren auf Basis von Avipox-, Raccoonpox, Capripox, Swinepox oder Vaccinia Virus sind als Vektoren zur Expression von fremder genetischer Information bereits beschrieben. Die dabei gewonnenen Erkenntnisse können nicht auf PPV übertragen werden. Wie vergleichende Untersuchungen gezeigt haben, gibt es morphologische, strukturelle und genetische Unterschiede zwischen den einzelnen Gattungen der Pockenviren. So lassen sich beipielsweise die PPV mit serologischen Methoden von den anderen Gattungen der Pockenviren unterscheiden, was auf unterschiedliche Proteinmuster und damit verbunden auf unterschiedliche Erbinformationen zurückzuführen ist. Einige Vetreter der Pockenviren haben beispielsweise die Fähigkeit zur Agglutination von Erythrozyten. Diese Aktivität wird über ein Oberflächenprotein vermittelt, das sogenannte Haemagglutinin (HA). PPV haben diese Aktivität nicht.

Die Kenntnisse über die Organisation des Genoms von PPV beschränken sich derzeit auf die Bestimmungen der Größe des Genoms, den GC-Gehalt der Nukleinsäure, vergleichende Restriktionsenzym-Analysen, die Klonierung einzelner Genomfragmente, Sequenzanalysen von Teilbereichen und die damit verbundene, präliminäre Beschreibung von einzelnen Genen (zur Übersicht Lit. #1, Lit. #5, Lit. #6).

Bei Vaccinia bekannte Insertionsstellen lassen sich aufgrund ihrer fehlenden oder nicht nachgewiesenen Existenz bei PPV derzeit nicht nutzen.

So brachten z.B. die Versuche, das Gen für Thymidinkinase im Genom des PPV zu identifizieren und, wie bei Orthopockenviren als Insertionsstelle zu nutzen, keine Ergebnisse. Mazur und Mitarbeiter (Lit. #3) beschreiben zwar die Identifizierung eines Genomabschnittes von PPV, der Ähnlichkeiten mit dem Thymidinkinase-Gen von Vaccinia Virus (ein Orthopockenvirus) haben soll, eigene umfangreiche Untersuchungen konnten allerdings die Existenz eines solchen Gens bei PPV nicht bestätigen. Auch andere Autoren (Lit. #1) konnten ein Thymidinkinase-Gen bei PPV nicht finden. Bei Vaccinia Virus wird das Gen für HA als Insertionsstelle für Fremd-DNA benutzt. PPV haben diese Aktivität, wie oben beschrieben, nicht.

Robinson und Lyttle erwähnten 1992 alternative Insertionstellen auf dem Genom von PPV (Lit. #1), ohne jedoch eine Beschreibung oder genaue Charakterisierung dieser Stellen zu gegeben. Auch erfolgte bis heute noch keine Beschreibung einer erfolgreichen Anwendung von PPV als Vektoren.

Bei den eigenen Untersuchungen zur Sequenzanalyse des HindIII-Fragmentes I von PPV Stamm D1701 wurde ein ORF mit Aminosäurehomologie (36,1 bis 38,3% Identität; 52,8 bis 58,6% Ähnlichkeit, GCG, Wisconsin Package 8.1, z.B. Programm Pikup) zum Vaskulären Endothelialen Wachstumsfaktor (VEGF) verschiedener Säugerspezies (z.B. Maus, Ratte, Meerschweinchen, Rind, Mensch) gefunden. Seq. ID No: 1 zeigt die Nukleotidsequenz des Gens bei D1701, Seq. ID No: 15 die Aminosäuresequenz des entsprechenden Proteins von D1701. Kürzlich wurde ein homologes Gen auch bei den PPV Stämmen NZ2 und NZ7 beschrieben (Lit. #6), dessen Funktion aber nicht bekannt ist. Ein entsprechendes Gen ist bei anderen Pockenviren, z.B. Orthopockenviren, nicht bekannt. Im weiteren Verlauf des Textes wird dieses Gen als VEGF-Gen bezeichnet.

Unsere Sequenzanalyse des HindIII-Fragmentes I von D1701 führte zur Identifizierung eines weiteren ORF, der Homologie zu Proteinkinase-Genen von Orthopockenviren hat und bei Vaccinia als F10L bekannt ist. Die Identität zum Vaccinia F10L-Gen beträgt 51%, die Ähnlichkeit 70%. Im weiteren Verlauf der Anmeldung wird dieses Gen PK-Gen genannt. Seq. ID No: 2, No: 9, No: 13 zeigt Versionen der Nukleotidsequenz des Gens bei D1701, Seq. ID No: 14 die Aminosäuresequenz des entsprechenden Proteins von D1701.

Es wurde weiterhin ein ORF gefunden, der das 3'-Ende des PK-Gens und das 5'-Ende des VEGF-Gens überlappt. Homologie-Untersuchungen zeigten eine geringe Identität (28%) und geringe Ähnlichkeit (51%) zum F9L-Gen bei Vaccinia. Seq. ID No: 5, No: 10, zeigt Versionen der Nukleotidsequenz des Gens bei D1701. Im weiteren Verlauf des Textes wird dieses Gen das F9L-Gen genannt.

Innerhalb der ITR-Region wurde ein weiterer ORF gefunden, der aufgrund seiner Ähnlichkeit zu einem Gen bei PPV NZ2 (Identität 76%, Ähnlichkeit 83%) mit ORF3 bezeichnet wird. Seq. ID No: 4 zeigt die Nukleotidsequenz des Gens bei D1701. Im weiteren Verlauf des Textes wird dieses Gen mit ORF3-Gen bezeichnet.

Gegenstand der vorliegenden Erfindung sind:
1. Rekombinant hergestellte PPV mit Insertionen und/oder Deletionen, enthaltend ein Fremdantigen, welches einen erregerspezifischen Schutz induzieren kann.
2. Rekombinant hergestellte PPV mit Insertionen und/oder Deletionen, enthaltend ein Zytokin.
3. Rekombinant hergestellte PPV gemäss 1 oder 2, enthaltend Insertionen und/oder Deletionen in Genomabschnitten, die nicht für die Virusvermehrung notwending sind.
4. Rekombinant hergestellte PPV gemäss 1 oder 2, enthaltend Insertionen und/oder Deletionen in Genomabschnitten, die für die Virusvermehrung notwending sind.

Die vorstehend aufgeführten Begriffe haben folgende Bedeutung:
- Attenuierung ist ein
   Prozeß bei dem die PPV durch Veränderung in ihrem Genom vermindert- oder nicht-pathogen bzw. -virulent für Tiere oder Menschen geworden sind.
- Deletionen sind
   fehlende DNA-Teilstücke aus dem Genom von PPV.
- Deletionsplasmide sind
   Plasmide, die neben der Plasmid-DNA PPV-Genomabschnitte tragen, denen Teilstücke entfernt wurden.
- Zur Virusvermehrung notwendige (essentielle) Genomabschnitte sind
   Teile des Gesamt-Genoms von PPV, die zur in vitro-Vermehrung von PPV, d.h. zur Bildung von infektiösen Virusnachkommen unverzichtbar sind.
   Eine Störung von Genen, die für die Virusvermehrung essentiell sind, führt zu einer Unterbrechnung in der Virusvermehrung. Entfernt man beispielsweise Teile eines dieser Gene oder das gesamte Gen, bricht die Virusreplikation an einer bestimmten Stelle im Vermehrungszyklus des Virus ab. Infektionen oder Behandlungen mit solchen Mutanten führen nicht zu einer Freisetzung infektiöser Nachkommen aus dem Tier. Ersetzt man Teile eines oder ein gesamtes essentielles Gene(s) durch Fremd-DNA oder inseriert Fremd-DNA in essentielle Gene, können Vektorvakzinen konstruiert werden, die im Impfling nicht vermehrungsfähig sind und somit nicht als infektiöse Erreger ausgeschieden werden.
- Zur Virusvermehrung nicht-notwendige (nicht-essentielle) Genomabschnitte sind
   Teile des Gesamt-Genoms von PPV, die zur in vitro-Vermehrung von PPV, d.h. zur Bildung von infektiösen Virusnachkommen, verzichtbar sind.
- Fremde DNA-Elemente (Fremd-DNA) sind
   DNA-Teilstücke, z.B. Fremd-Gene oder Nukleotidsequenzen, die in dem erfindungsgemäß eingesetzten PPV ursprünglich nicht vorhanden sind.
   Fremd-DNA wird aus folgenden Gründen in das PPV inseriert:
   1. zur Expression der Fremd-DNA
   2. zur Inaktivierung von Funktionen von DNA-Teilstücken des PPV
   3. zur Markierung des PPV.

   In Abhängigkeit von diesen Gründen wird unterschiedliche Fremd-DNA inseriert. Soll gemäß (1) Fremd-DNA exprimiert werden, so wird die inserierte Fremd-DNA mindestens einen offenen Leserahmen tragen, der für ein oder mehrere gewünschte Fremdprotein(e) kodiert. Gegebenenfalls enthält die Fremd-DNA zusätzlich eigene oder fremde Regulatorsequenzen. Die Kapazität zur Aufnahme von Fremd-DNA kann durch Setzen von Deletionen im Genom des Virus vergrößert werden. Im allgemeinen liegt die Länge zwischen 1-Nukleotid und 35.000, bevorzugt zwischen 100 und etwa 15.000 Nukleotiden.
   Als Beispiele seien genannt, Gene oder Genteile von Viren, wie beispielsweise
   Herpesvirus suid 1,
   Equine Herpesviren
   Bovine Herpesviren
   Maul- und Klauenseuche-Virus,
   Bovines Respiratorisches Syncytial Virus,
   Parainfluenzavirus 3 des Rindes,
   Influenzavirus,
   Calicivirus
   Flaviviren, z.B. Bovines Virus Diarrhoe Virus oder Virus der klassischen Schweinepest
   oder von Bakterien, wie beispielsweise
   Pasteurella spec.;
   Salmonella spec.,
   Actinobacillus spec.,
   Chlamydia spec.,
   oder von Parasiten, wie beispielsweise
   Toxoplasma,
   Dirofilaria,
   Echinococcus.

   Soll gemäß (2) Fremd-DNA inseriert werden, reicht für die Unterbrechung der Vektor-Virus-DNA-Sequenz prinzipiell die Insertion eines geeigneten Fremdnukleotids. Die maximale Länge der zur Inaktivierung inserierten Fremd-DNA richtet sich nach der Aufnahmekapazität des Vektor-Virus für Fremd-DNA. Im allgemeinen ist die Länge der Fremd-DNA zwischen 1 Nukleotid und 35.000 Nukleotiden, bevorzugt zwischen 100 und 15.000 Nukleotiden, besonders bevorzugt zwischen 3 bis 100 Nukleotiden.
   Sollen gemäß (3) DNA-Sequenzen zur Markierung inseriert werden, richtet sich ihre Länge nach der Nachweismethode zur Identifizierung des markierten Virus. Im allgemeinen ist die Länge der Fremd-DNA zwischen 1 und 25.000 Nukleotiden, bevorzugt zwischen 20 Nukleotiden und 15.000 Nukleotiden, besonders bevorzugt zwischen 5 bis 100 Nukleotiden.
- Genbank
   ist die Gesamtheit von in vermehrungsfähigen Vektoren enthaltenen Fragmente eines Genoms. Sie wird durch Fragmentierung des Genoms und Insertion aller, einzelner oder eines Großteils der Fragmente in vermehrungsfähige Vektoren, wie z.B. Plasmide, erhalten.
- Genomfragment ist
   ein Teilstück eines Genoms, das isoliert vorliegen oder in einen vermehrungsfähigen Vektor inseriert sein kann.
- Inaktivierung durch Insertion bedeutet,
   daß die inserierte Fremd-DNA die Expression oder Funktion PPV-eigener Genom-Sequenzen verhindert.
- Insertionen sind
   DNA-Teilstücke, die zusätzlich in das Genom von PPV eingebaut wurden. Die Länge der DNA-Teilstücke kann je nach Grund der Insertion zwischen 1 Nukleotid und mehreren tausend Nukleotiden liegen (siehe auch Definition für "Fremd-DNA").
- Insertionsplasmide sind
   Plasmide, insbesondere bakterielle Plasmide, die die zu inserierende Fremd-DNA, flankiert von DNA-Sequenzen des PPV, beinhalten.
- Insertionsstellen sind
   Stellen in einem Virus-Genom, die zur Aufnahme von Fremd-DNA geeignet sind.
- Klonierung heißt,
   daß die genomische DNA von PPV isoliert und fragmentiert wird. Die Fragmente oder eine Auswahl der Fragmente werden in gängige DNA-Vektoren (bakterielle Plasmide oder Phagenvektoren oder eukaryontische Vektoren) inseriert.
   Eine Auswahl an Methoden zur Herstellung und Klonierung von DNA-Fragmenten gibt Lit. #11. Die DNA-Vektoren mit den PPV-DNA-Fragmenten als Inserts dienen z.B. der Herstellung von identischen Kopien der ursprünglich isolierten DNA-Fragmente von PPV.
- Markierung durch Insertion bedeutet,
   daß die inserierte Fremd-DNA die spätere Identifizierung des veränderten PPV ermöglicht.
- Unter ORF (Open Reading Frame) versteht man eine Abfolge von Nukleotiden auf DNA-Ebene, die die Aminosäuresequenz eines potentiellen Proteins definiert. Sie besteht aus einer durch die Größe des von ihr definierten Proteins bestimmten Anzahl von Nukleotid-Tripletts, die 5'-seitig durch ein Startcodon (ATG) und 3'-seitig durch ein Stopcodon (TAG, TGA, TAA) begrenzt wird.
- Regulatorsequenzen sind
   DNA-Sequenzen, die die Expression von Genen beeinflussen. Solche sind bekannt aus Lit. #15.
   Bevorzugt sei genannt der VEGF-Promotor, wie er in Sequenz Protokoll ID No: 6 beschrieben ist.
- Rekombinante PPV sind
   PPV mit Insertionen und/oder Deletionen in ihrem Genom. Die Insertionen und Deletionen wurden hierbei über molekularbiologische Methoden hergestellt.
- Repetitive (DNA)-Sequenzen sind
   identische Nukleotid-Sequenzen, die im PPV-Genom direkt nacheinander oder verstreut an unterschiedlichen Stellen vorkommen.
- Vektor-Virus ist
   ein PPV, das zur Insertion von Fremd-DNA geeignet ist und die inserierte Fremd-DNA in seinem Genom in infizierte Zellen oder Organismen transportieren kann und gegebenenfalls die Expression der Fremd-DNA ermöglicht.

Die Herstellung der erfindungsgemäßen PPV erfolgt wie folgt beschrieben:
- 1.: Auswahl eines geeigneten PPV-Stammes
- 2.: Identifizierung von Genomabschnitten mit Insertionsstellen in dem Genom des PPV
- 2.a: Identifizierung von PPV-Genomabschnitten mit Insertionsstellen in Genen, die nicht-essentiell für die Virusvermehrung sind,
- 2.b: Identifizierung von PPV Genomabschnitten mit Insertionsstellen in Genen, die essentiell für die Virusvermehrung sind,
- 2.c: Identifizierung von Genomabschnitten mit Insertionsstellen in Bereichen außerhalb von Genen in dem Genom des PPV und/oder in Genduplikationen
- 2.d: Weitere Methoden zur Identifizierung von Genomabschnitten mit Insertionsstellen
- 2.e: Anforderungen an eine Insertionsstelle
- 2.1: Identifizierung von Insertionsstellen
- 2.1.1: Reinigung des Genoms von PPV
- 2.1.2: Klonierung der Genomfragmente, Etablierung einer Genbank
- 2.1.3: Sequenzierung zur Identifizierung von Genen oder Genomabschnitten außerhalb von Genen
- 2.1.4: Auswahl der Klone mit PPV-Genomfragmenten zur Weiterverarbeitung
- 2.2: ITR-Region, VEGF-, PK-Gen, Gen kodierend für das 10 kDa-Protein und der Bereich zwischen dem PK- und dem HDIR-Gen als Insertionsstellen
- 2.2.1: Klonierung des VEGF-Gens
- 2.2.2: Klonierung des Proteinkinase-Gens
- 2.2.3: Klonierung des Gen-Bereichs der für das 10kDa-Protein kodiert
- 2.2.4: Klonierung der ITR-Region (Inverted-Terminal-Repeat-Region) bzw. des Genomabschnittes, der zwischen dem PK-Gen und dem HD1R-Gen liegt
- 3.: Konstruktion von Insertionsplasmiden oder von Deletionsplasmiden, die die zu inserierende Fremd-DNA enthalten,
- 3.1: Identifizierung oder Herstellung von nur einmal vorkommenden, d.h. singulären Restriktionsenzymerkennungsstellen ("unique restriction sites") in den klonierten Genomfragmenten und Insertion von Fremd-DNA
- 3.2: Deletion von Genomsequenzen in den klonierten Genomfragmenten und Insertion von Fremd-DNA
- 3.3: Eine Kombination von #3.1 und #3.2
- 4.: Konstruktion eines rekombinanten PPV

### 1. Auswahl eines geeigneten PPV-Stammes

Grundsätzlich sind alle PPV-Spezies für die Durchführung der vorliegenden Erfindung geeignet. Bevorzugt werden Virusstämme, die sich zu Titern > 10⁵ PFU (Plaque Forming Unit)/ml in einer Gewebekultur vermehren lassen und aus dem Medium der infizierten Zellen als extrazelluläres, infektiöses Virus rein darstellen lassen. Bevorzugt genannt sei vom Genus der PPV die Spezies PPV ovis (Orf Viren).

Als besonders bevorzugt genannt sei der PPV-ovis-Stamm D1701, hinterlegt nach dem Budapester Vertrag am 28.04.1988 bei Institut Pasteur, C.N.C.M. unter der Reg.Nr. CNCM I-751, sowie seine Varianten und Mutanten.

Die Vermehrung der Viren erfolgt in üblicher Weise in Gewebekulturen animaler Zellen wie Säugetierzellen, z.B. in Schafzellen, Rinderzellen, bevorzugt in Rinderzellen wie der permanenten Rindernierenzelle BK-K1-3A (oder deren Abkömmlingen) oder Affenzellen wie den permanenten Affennierenzellen MA104 oder Vero (oder deren Abkömmlingen).

Die Vermehrung erfolgt in an sich bekannter Weise in stationären, Roller- oder Carrier-Kulturen in Form von geschlossenen Zellverbänden oder in Suspensionskulturen.

Die zur Vermehrung der Viren dienenden Zellen und Zellrasen werden in üblicher Weise nahezu bis zur Konfluenz oder bis zu optimalen Zelldichte vermehrt. Die Zellen werden mit Virusverdünnungen infiziert, die einer MOI (= multiplicity of infection, entspricht infektiöse Viruspartikel pro Zelle) entsprechen.

Die Vermehrung der Viren erfolgt mit oder ohne Zusatz von Tierseren. Für den Fall, daß Serum eingesetzt wird, wird dieses zum Vermehrungsmedium in einer Konzentration von 1-30 Vol.-%, vorzugsweise 1-10 Vol.-% zugegeben.

Infektion und Virusvermehrung erfolgt bei Temperaturen zwischen Raumtemperatur und 40° C, bevorzugt zwischen 32 und 39° C, besonders bevorzugt bei 37° C über mehrere Tage, bevorzugt bis zur vollständigen Zerstörung der infizierten Zellen. Bei der Virusernte kann noch zellgebundenes Virus mechanisch oder durch Ultraschall oder milde enzymatische Proteolyse z.B. mit Trypsin zusätzlich freigesetzt werden.

Das virushaltige Medium der infizierten Zellen kann dann weiter aufgearbeitet werden, z.B. durch Entfernung der Zelltrümmer mittels Filtration mit Porengrößen von z.B. 0,2-0,45 µm und/oder niedertourige Zentrifugation.

Filtrat oder Zentrifugationsüberstand können zur Virusanreicherung und - reinigung verwendet werden. Dazu werden Filtrat oder Überstand einer hochtourigen Zentrifugation bis zur Sedimentation der Viruspartikel unterworfen. Gegebenenfalls können weitere Reinigungsschritte durch z.B. Zentrifugation in einem Dichtegradienten angeschlossen werden.

### 2. Identifizierung von Genomabschnitten mit Insertionsstellen im Genom des PPV

Verschiedene Bereiche des Genoms des PPV können als Insertionsstellen zur Insertion von Fremd-DNA dienen. Fremd-DNA kann inseriert werden,
a. in Gene, die nicht-essentiell für die Virusvermehrung in vitro und/oder in vivo sind, .
b. in Gene, die essentiell für die Virusvermehrung sind und/oder
c. in Bereichen ohne Genfunktion.

### 2.a Identifizierung von Genomabschnitten mit Insertionsstellen in Genen, die nicht-essentiell für die Virusvermehrung sind, im Genom des PPV

i. Virale Gene, die nicht-essentiell für die Virusvermehrung sind, werden beispielsweise durch vergleichende Untersuchungen mit Vertretern verschiedener PPV-Spezies gefunden. Gene, die in einem oder mehreren Isolaten oder Stämmen einer PPV-Spezies nicht vorkommen, in anderen Isolaten oder Stämmen aber gefunden werden, sind potentiell nicht-essentiell.
ii. Gene, die nicht-essentiell für die Virusvermehrung sind, können auch auf einem alternativen Weg identifiziert werden. Nach der Sequenzierung von Genomfragmenten von PPV, die beispielsweise als klonierte Fragmente vorliegen können, werden diese DNA-Sequenzen auf mögliche "Offene Leserahmen" ("Open Reading Frames", ORF) untersucht. Findet man ein ORF, wird dessen Funktion als Gen über den Nachweis der Transkription und/oder Translation verifiziert. Um festzustellen, ob das gefundene Gen nicht-essentiell für die Virusvermehrung ist, wird das Gen durch molekulargenetische Methoden aus dem Genom des PPV entfernt, partiell zerstört oder durch eingeführte Mutation(en) unterbrochen, und das resultierende Virus auf Vermehrungsfähigkeit untersucht. Kann das Virus auch ohne die Existenz des manipulierten Genes replizieren, handelt es sich hierbei um ein nicht-essentielles Gen.

### Beispiele für identifizierte Insertionsstellen

i. Als Beispiel für ein nicht-essentielles Gen von PPV sei hier das VEGF-Gen des PPV ovis genannt, das als Inertionsstelle für Fremd-DNA dient. Dieses Gen kommt in den untersuchten Stämmen von Parapoxvirus ovis (NZ-2, NZ-7 und D1701 vor (Lit. #6)). Bei einigen PPV-Stämmen, beispielsweise Vertreter von PPV bovis 1 ist dieses VEGF-Gen nicht nachgewiesen. Die Identifizierung des Bereiches mit dem VEGF-Gen auf dem Genom von PPV kann mit Hilfe der im Sequenz Protokoll ID No: 1 gezeigten DNA-Sequenz erfolgen. Über die üblichen Methoden der Molekularbiologie kann das Gen mittels Hybridisierungsexperimenten, Genom-Sequenzanalysen und/oder Polymerase-Ketten-Reaktionen auf dem Genom eines PPV gefunden werden.
ii. Als weiteres Beispiel für ein potentiell nicht-essentielles Gen von PPV sei das Gen für das 10kDa-Protein von PPV genannt. Dieses Gen kommt in Stämmen von Parapoxvirus ovis (NZ-2, NZ-7 und D1701) vor. Die Identifizierung des Bereiches mit dem Gen für das 10kDa-Protein auf dem Genom von PPV kann mit üblichen Methoden der Molekularbiologie wie beispielsweise mit Hilfe der Polymerase-Ketten-Reaktionen (PCR) auf dem Genom eines PPV gefunden werden. Lit. #8 zeigt die DNA-Sequenz des 10kDa-Protein-Gens. Die für eine PCR einsetzbaren Primer sind beispielsweise in Lit. #8 genannt. Die DNA-Sequenz des 10kDa-spezifischen PCR-Produktes von D1701 zeigt Sequenzprotokoll ID No: 11.

Für die Insertion von Fremd-DNA kann das nicht-essentielle Gen in Teilen oder vollständig aus dem Genom des PPV entfernt werden. Es ist aber auch möglich, Fremd-DNA in das nicht-essentielle Gen zu inserieren, ohne daß Bereiche des PPV-Gens entfernt werden. Als Insertionsstellen können beispielsweise Restriktionsenzym-Erkennungsstellen dienen.

### 2.b Identifizierung von PPV-Genomabschnitten mit Insertionsstellen in Genen, die essentiell sind, auf dem Genom des PPV

i. Die Identifizierung essentieller Gene kann durch Sequenzierung von viralen Genomfragmenten die beispielsweise als klonierte Fragmente vorliegen, und Identifizierung möglicher ORF erfolgen.
   Findet man einen ORF, wird dessen Funktion als Gen über den Nachweis der Transkription und/oder Translation verifiziert. Um festzustellen, ob das gefundene Gen essentiell für die Virusvermehrung ist, wird das Gen durch molekulargenetische Methoden im Genom des PPV zerstört, beispielsweise durch Entfernung von Teilen oder des gesamten Gens oder durch Insertion von Fremd-DNA, und das resultierende Virus auf Vermehrungsfähigkeit untersucht. Kann die erhaltene Virusmutante nicht replizieren, handelt es sich hierbei mit großer Wahrscheinlichkeit um ein essentielles Gen.

Beweisend ist das ausschließliche Wachstum der Virusmutante in komplementierenden Zellinien.

### Beispiele für Insertionsstellen

Als Beispiel sei das Proteinkinasegen (PK-Gen) von PPV D 1701 genannt. Das PK-Gen wird spät im Vermehrungszyklus des Virus exprimiert. Die Identifizierung des PK-Gens auf dem Genom von PPV kann mit Hilfe der im Sequenz Protokoll ID No: 2, No: 9 oder No: 13 gezeigten Versionen der DNA-Sequenz erfolgen. Über die üblichen Methoden der Molekularbiologie kann der Bereich mit dem Gen beispielsweise durch Hybridisierungsexperimente, Genom-Sequenzanalysen und/oder Polymerase-Ketten-Reaktionen auf dem Genom eines PPV gefunden werden.

Für die Insertion von Fremd-DNA kann das essentielle Gen in Teilen oder vollständig aus dem Genom des PPV entfernt werden. Es ist aber auch möglich, die Fremd-DNA in das essentielle Gen zu inserieren, ohne daß Bereiche aus dem PPV-Gen entfernt werden.

### 2.c Identifizierung von Genomabschnitten mit Insertionsstellen in Bereichen außerhalb von Genen auf dem Genom des PPV und/oder in Genduplikationen

Genomabschnitte, die nicht für funktionelle Genprodukte kodieren und keine essentiellen regulatorischen Funktionen besitzen (sog. intergenische Abschnitte), sind prinzipiell als Insertionsstellen für Fremd-DNA geeignet. Besonders geeignet sind Bereiche mit repetitiven Sequenzen, da Veränderungen in Teilbereichen durch verbleibende Sequenzrepetitionen kompensiert werden können. Hierunter fallen auch Gene, die in zwei- oder mehrfacher Kopie vorkommen, sogenannte Genduplikationen.

Gene in der ITR-Region oder in duplizierten Abschnitten des PPV-Genoms existieren in zwei Kopien im Virusgenom. Nach Entfernen oder Veränderung einer Kopie eines solchen Gens und Insertion von Fremd-DNA können stabile PPV Rekombinanten erhalten werden, auch wenn das geänderte Gen für die Virusvermehrung wichtig sein sollte. Eine zweite, ungeänderte Genkopie könnte für die Genfunktion ausreichen.
i. Die Identifizierung von Genomsequenzen, die nicht für Genprodukte kodieren, erfolgt über Sequenzanalysen des Genoms der PPV. Genombereiche, die weder nach Sequenzanalyse einen ORF noch virusspezifische Transkription zeigen und keine regulatorische Funktion aufweisen, stellen potentielle Insertionsstellen dar. Besonders die Schnittstellen für Restriktionsenzyme in diesen Bereichen repräsentieren potentielle Insertionsstellen. Zur Überprüfung, ob eine geeignete Insertionsstelle vorliegt, wird über bekannte molekularbiologische Methoden Fremd-DNA in die potentielle Insertionsstelle inseriert und die resultierende Virusmutante auf Lebensfähigkeit untersucht. Wenn die Virusmutante, die in der möglichen Insertionstelle Fremd-DNA trägt, vermehrungsfähig ist, handelt es sich bei der untersuchten Stelle um eine geeignete Insertionstelle.
ii. Die Identifizierung von repetitiven Sequenzen sowie Genduplikationen erfolgt beispielsweise über DNA-Hybridisierungsexperimente und/oder über Sequenzanalysen. Bei den Hybridisierungsexperimenten werden klonierte oder isolierte Genomfragmente eines PPV als Sonden für Hybridisierungen mit PPV-DNA-Fragmenten eingesetzt. Die Genomfragmente des PPV, die mit mehr als einem Fragment des Gesamtgenoms des PPV hybridisieren, enthalten eine oder mehrere repetitive Sequenzen. Um die repetitive Sequenz oder duplizierte Genombereiche auf dem Genom-Fragment genau zu lokalisieren, wird die Nukleotidsequenz bestimmt. Um festzustellen, ob eine potentielle Insertionsstelle eine geeignete Insertionsstelle im Gesamtgenom des PPV ist, muß Fremd-DNA in eine repetitive Sequenz oder in eine Kopie der Genduplikation inseriert werden und das PPV-Genom-Fragment mit dem Insert in das Virusgenom eingebaut werden. Anschließend wird das Fremd-DNA enthaltende rekombinante Virus auf Vermehrungsfähigkeit geprüft. Vermehrt sich das rekombinante Virus, ist die identifizierte Erkennungsstelle als Insertionsstelle geeignet.

### Beispiele für Insertionsstellen

i. Als Beispiele für intergenische Bereiche seien der Genomabschnitt zwischen dem Gen für die Proteinkinase und dem HDIR-Gen (Sequenz Protokoll ID No: 7) genannt.
ii. Als Beispiel für eine repetitive Sequenz sei die ITR-Region genannt (Sequenz Protokoll ID No: 4).
iii. Als Beispiel einer Genduplikation im PPV-Stamm D1701 sei das potentielle Gen "ORF 3" (in der ITR-Region) sowie das VEGF-Gen genannt. Hybridisierungsstudien bewiesen, daß im vorliegenden Stamm D1701 ein Bereich, der das VEGF-Gen beinhaltet, dupliziert und an das andere Ende des Virusgenoms transloziert wurde, so daß zwei VEGF-Genkopien vorliegen.
   Anhand der Sequenzen im Sequenz Protokoll ID No: 4 (ITR-Sequenz mit Gen "ORF3") und ID No: 7 (Bereich zwischen PK-und HDIR-Gen) können die entsprechenden Genombereiche bei anderen PPV mit üblichen Methoden der Molekularbiologie, beispielsweise Hybridisierungsexperimenten, Genom-Sequenzanalysen und/oder Polymerase-Ketten-Reaktionen gefunden werden.

### 2.d Weitere Methoden zur Identifizierung von Insertionstellen

Generell werden mögliche Insertionsstellen auf dem Genom von PPV auch über Modifikationen der viralen Genomsequenzen gefunden. Genomstellen, an denen Nukleotid-Austausche, Deletionen und/oder Insertionen oder Kombinationen hieraus, die Virusvermehrung nicht blockieren, stellen mögliche Insertionsstellen dar. Zur Überprüfung, ob eine potentielle Insertionsstelle eine geeignete Insertionsstelle darstellt, wird über bekannte molekularbiologische Methoden Fremd-DNA in die potentielle Insertionsstelle inseriert und die resultierende Virusmutante auf Lebensfähigkeit untersucht. Wenn die Virusrekombinante vermehrungsfähig ist, handelt es sich bei der untersuchten Stelle um eine geeignete Insertionstelle.

### 2.1 Identifizierung von Insertionsstellen

### 2.1.1 Reinigung des Genoms von PPV

Für die molekulargenetische Klonierung von Insertionsstellen von PPV wird zunächst das Genom der PPV gereinigt. Ausgehend von dem gemäß 1 (oben) hergestellten Virus wird das Genom isoliert und gereinigt. Die Extraktion von nativer viraler DNA erfolgt bevorzugt über die Behandlung der gereinigten Virionen mit wäßrigen Lösungen von Detergentien und Proteasen.

Als Detergentien seien genannt anionische, kationische, amphotere, nichtionische Detergentien. Bevorzugt werden ionische Detergentien eingesetzt. Besonders bevorzugt werden Natriumdodecylsulfat, Natriumlaurylsulfat.

Als Proteasen seien genannt alle Proteasen, die in Gegenwart von Detergens arbeiten, wie z.B. Proteinase K und Pronase. Bevorzugt sei genannt Proteinase K.

Detergentien werden in Konzentrationen von 0,1-10 Vol.-% eingesetzt, bevorzugt sind 0,5-3 Vol.-%.

Proteasen werden in Konzentrationen von 0,01-10 mg/ml Viruslysat eingesetzt, bevorzugt sind 0,05-0,5 mg/ml Viruslysat.

Es wird bevorzugt in wäßriger gepufferter Lösung in Gegenwart von DNase-Inhibitoren gearbeitet. Als Puffersubstanzen seien genannt: Salze schwacher Säuren mit starken Basen wie z.B. Tris(hydroxymethylaminomethan), Salze starker Säuren mit schwachen Basen wie z.B. primäre Phosphate oder Gemische derselben.

Bevorzugt sei das folgende Puffersystem genannt: Tris(hydroxymethylaminomethan).

Die Puffersubstanzen oder Puffersysteme werden in Konzentrationen eingesetzt, die pH-Werte gewährleisten, bei denen die DNA nicht denaturiert. Bevorzugt sind pH-Werte von 5-9, besonders bevorzugt 6-8,5 , ganz besonders bevorzugt 7-8, insbesondere sei Arbeiten im neutralen Bereich genannt.

DNase-Inhibitoren sind z.B. Ethylendiamintetraessigsäure in Konzentrationen von 0,1-10 mM (Millimol), bevorzugt ist ca. 1 mM.

Anschließend werden die lipophilen Bestandteile des Viruslysats extrahiert. Als Extraktionsmittel dienen Lösungsmittel wie Phenol, Chloroform, Isoamylalkohol oder deren Gemische. Bevorzugt wird zunächst ein Gemisch aus Phenol und Chloroform/Isoamylalkohol eingesetzt, wobei die Extraktion in einer oder mehreren Stufen erfolgt.

Weitere Methoden zur Isolierung der Virus-DNA sind z.B. Zentrifugation eines Viruslysats in einem CsCI-Dichtegradienten oder in der Gelelektrophorese (s. Lit. #14).

Die Extraktion von Nukleinsäuren wird in Lit. #13 beschrieben.

Die so extrahierte DNA wird bevorzugt aus der wäßrigen Lösung mit z.B. Alkohol, bevorzugt mit Ethanol oder Isopropanol und unter Zusatz von monovalenten Salzen wie z.B. Alkalichloride oder -acetate, bevorzugt Lithiumchlorid, Natriumchlorid oder Natriumacetat, Kaliumacetat, ausgefällt (s. loc cit.).

### 2.1.2 Klonierung der Genomfragmente

Die so gereinigte virale DNA dient nun der Herstellung von DNA-Fragmenten. Sie wird hierzu beispielsweise mit Restriktionsenzymen behandelt. Geeignete Restriktionsenzyme sind beispielsweise Eco RI, BamH1 HindIII, Kpnl. Alternativ können Genomfragmente mittels Polymerase-Kettenreaktion (PCR) synthetisiert werden. Hierzu werden Primer aus bereits bekannten Sequenzabschnitten des VirusGenoms ausgewählt und mittels beispielsweise Taq bzw. Pfu-Polymerase der vom Primerpaar begrenzte Genomabschnitt in vitro synthetisiert.

Die aus Restriktionsverdau bzw. PCR resultierenden DNA-Fragmente können nach den bei (Sambrock 89) beschriebenen Methoden in Vektorsysteme kloniert werden. Beispielsweise bieten sich hierzu, abhängig von der Größe des jeweilig zu klonierenden DNA-Fragmentes, Plasmidvektoren, Lambdaphagen-Vektoren, bzw. Cosmidvektoren an.

### 2.1.3 Sequenzierung. Identifizierung und Charakterisierung von Genen. Verifizierung ihrer Expression

In Vektoren klonierte Genomfragmente werden zunächst durch Sequenzierung analysiert. Die inserierten DNA-Fragmente werden mittels verschiedener Restriktionsenzyme kartiert und geeignete Subfragmente in Plasmidvektoren kloniert. Die Sequenzierungsreaktion erfolgt beispielsweise unter Verwendung des T7-Sequencing Kit der Firma Pharmacia nach Angaben des Herstellers. Die hierzu benötigte doppelsträngige Plasmid-DNA wird vorzugsweise nach der PEG-Methode (Hattori und Sakaki 1985) präpariert. Mittels Computeranalyse (CGC, s. vorne) werden auf den Genomfragmenten vorhandene "Offene Leserahmen (ORF)" identifiziert. Erkenntnisse über die jeweilige Funktion der identifizierten ORFs können durch Vergleich ihrer Sequenz mit anderen in einer Datenbank vorhandenen Gensequenzen bekannter Funktion gewonnen werden. Funktionell charakterisiert werden die identifizierten ORFs durch Nachweis ihrer jeweiligen korrespondierenden Transkripte in Virus-infizierten Zellen. Hierzu wird die Gesamt-RNA virus-infizierter Zellen beispielsweise mittels der AGPC-Methode (Chomczynski und Sacchi, (20) 1987) isoliert. Durch Northern Blotanalyse bzw. Primer-Extension und RNA-Schutzversuchen können die spezifischen Transkripte samt ihrer 5'- und 3'-Enden identifiziert werden. Alternativ besteht die Möglichkeit, das durch einen identifizierten ORF kodierte Virusprotein in vitro zu exprimieren, mittels des Expressionsproduktes Antiseren zu gewinnen und die Expression des ORFs nachzuweisen.

Um festzustellen, ob das gefundene Gen nicht-essentiell für die Virusvermehrung ist, kann das Gen durch Gendisruption bzw. Gendeletion zerstört werden. Hierbei werden entweder das gesamte Gen bzw. Teile davon aus dem PPV-Genom entfernt oder der Leserahmen des Gens durch die Insertion von Fremdgensequenzen unterbrochen. Das resultierende Virus wird auf Vermehrungsfähigkeit untersucht. Kann das Virus auch ohne die Existenz des zerstörten Genes replizieren, handelt es sich hierbei um ein nicht-essentielles Gen.

### 2.1.4 Auswahl der Klone mit PPV-Genomfragmenten

Welche der oben erhaltenen Klone mit Genomfragmenten des PPV eingesetzt werden, hängt davon ab, ob die herzustellenden rekombinanten PPV (i) replikationsfähig oder (ii) replikationsdefekt sein sollen.
i. Sollen rekombinante PPV hergestellt werden, die trotz der Insertion und/oder Deletion replikationsfähig sind, werden klonierte virale Genomfragmente weiterverarbeitet, die Gene oder Genombereiche außerhalb von Genen enthalten, die nicht-essentiell für die Virusvermehrung sind oder Genduplikationen enthalten.
   Die Prüfung, ob es sich bei dem vorliegenden Gen oder Genombereich um eine nicht-essentielle Region des Virusgenoms oder um eine Genduplikation handelt, wird über Virusmutanten bestimmt. Hierzu wird das untersuchte Gen bzw. der untersuchte Genombereich im PPV durch molekularbiologische Methoden inaktiviert, beispielsweise durch teilweise oder vollständige Deletion des fraglichen Bereiches, und die Vermehrungsfähigkeit der Virusmutante untersucht. Kann die Virusmutante sich trotz der Inaktivierung des fraglichen Gens oder Genombereiches vermehren, handelt es sich bei dem untersuchten Gen oder Genombereich um eine nicht-essentielle Region.
   Bevorzugt werden klonierte Genomfragmente des PPV, die die nicht-essentiellen Gene vollständig enthalten. Darüber hinaus sollten an beiden Enden der Gene oder der Genombereiche die flankierenden viralen Genombereiche ebenfalls enthalten sein. Die Länge der flankierenden Bereiche sollte mehr als 100 Basenpaare betragen. Liegen solche Genomklone nicht vor, können sie aus bestehenden Genomklonen mit molekularbiologischen Methoden hergestellt werden. Enhalten die klonierten Genomfragmente zusätzliche Genombereiche, die für die vorliegende Herstellung nicht benötigt werden, können diese durch Subklonierungen entfernt werden.
ii. Sollen rekombinante PPV hergestellt werden, die aufgrund der Insertion und/oder Deletion die Fähigkeit zur Bildung von infektiösen Nachkommen verloren haben, werden klonierte virale Genomfragmente weiterverarbeitet, die Gene oder Genombereiche außerhalb von Genen enthalten, die essentiell für die Virusvermehrung sind.
   Die Prüfung, ob es sich bei dem vorliegenden Gen oder Genombereich um eine essentielle Region des Virusgenoms handelt, kann über Virusmutanten bestimmt werden. Hierzu wird das untersuchte Gen bzw. der untersuchte Genombereich im PPV durch molekularbiologische Methoden inaktiviert, beispielsweise durch teilweise oder vollständige Deletion des fraglichen Bereiches, und die Vermehrungsfähigkeit der Virusmutante untersucht. Kann die Virusmutante sich aufgrund der Inaktivierung des fraglichen Gens oder Genombereiches nicht mehr vermehren, handelt es sich bei dem untersuchten Gen oder Genombereich um eine essentielle Region.
   Bevorzugt werden klonierte Genomfragmente des PPV, die die essentiellen Gene vollständig enthalten. Darüber hinaus sollten an beiden Enden der Gene oder der Genombereiche die flankierenden viralen Genombereiche ebenfalls enthalten sein. Die Länge der flankierenden Bereiche sollte mehr als 100 Basenpaare betragen. Liegen solche Genomklone nicht vor, können sie aus bestehenden Genomklonen mit molekularbiologischen Methoden hergestellt werden. Enhalten die klonierten Genomfragmente zusätzliche Genombereiche, die für die vorliegende Herstellung nicht benötigt werden, können diese durch Subklonierungen entfernt werden.

### 2.2 ITR-Region VEGF-Gen, PK-Gen, Gen kodierend für das 10kDa-Protein und der Bereich zwischen dem PK-Gen und dem HD1R-Gen als Insertionsstellen

Soll die ITR-Region, das VEGF-Gen, das PK-Gen, das Gen, das für 10kDa-Protein kodiert oder der intergenische Bereich zwischen dem PK-Gen und dem HD1R-Gen als Insertionsstelle in einem PPV benutzt werden, müssen die entsprechenden Bereiche des PPV-Genoms, die die Insertionsstellen beinhalten, isoliert werden. Hierzu werden die entsprechenden Bereiche des PPV-Genoms kloniert.

### 2.2.1 Klonierung des VEGF-Gens

Das Gen, das für VEGF kodiert, wird auf dem Genom des PPV lokalisiert und anschließend in Teilen oder in seiner Gesamtheit mit seinen flankierenden Genom-Abschnitten isoliert. Hierzu wird bevorzugt das PPV gemäß #1 vermehrt und das Genom gemäß #2.1.1 gereinigt.
a. Das VEGF-Gen wird beispielsweise über eine Polymerase-KettenReaktion (PCR) amplifiziert. Die für diese Reaktion notwendigen Start-Sequenzen (Primer) werden aus der im Sequenz Protokoll ID No: 1 dargestellten DNA-Sequenz des VEGF-Gens abgeleitet. Das erhaltene Amplifikat wird bevorzugt anschließend kloniert.
b. Bevorzugt wird die Region, die das VEGF-Gen und seine flankierenden Genomabschnitte enthält, über Fragmentierung des PPV-Genoms und Isolierung sowie Klonierung des oder der entsprechenden Genom-Fragmente gewonnen. Hierfür wird das gereinigte Genom des Virus wie in #2.1.2 beschrieben, bevorzugt mit dem Restriktionsenzym HindIII, geschnitten. Zur Identifizierung des oder der Genomfragmente, das oder die das VEGF-Gen und seine flankierenden Genomabschnitte tragen, werden bevorzugt die nach dem Enzymverdau erhaltenen Genom-Fragmente durch elektrophoretische oder chromatographische Verfahren aufgetrennt.
   Elektrophoretische Auftrennungen in Agarose bzw. Polyacrylamid werden nach Standardmethoden durchgeführt, die in
   - Current Protocols in Molecular Biology 1987-1988, Verlag Wiley-Interscience,1987
   - A Practical Guide to Molecular Cloning, Perbal, 2^{nd} edition, Verlag Wiley Interscience,1988
   - Molecular Cloning, loc. cit.
   - Virologische Arbeitsmethoden, Band III, Gustav Fischer Verlag, 1989
   beschrieben sind.
   Die Identifizierung der Genom-Fragmente, die das VEGF-Gen und seine flankierenden Sequenzen tragen, erfolgt beispielsweise durch Hybridisierung mit definierten Nukleinsäuresonden. Die aufgetrennten Genomfragmente werden hierzu auf Filter übertragen und mit VEGF-spezifischen und markierten Nukleinsäure-Sonden nach der Southern Blotmethode hybridisiert. Die Methode des Transfers der Genomfragmente und der Hybridisierung kann nach Standard-Protokollen, wie sie unter "Southern Blotting" in Molecular Cloning loc.cit. beschrieben sind, erfolgen. Die als Sonden einsetzbaren Oligonukleotide oder Nukleinsäurefragmente lassen sich aus Sequenzprotokoll Seq ID No: 1 ableiten. Beipielsweise wird das TaqI-Subfragment (366 bp), das mittels Seq ID No: 1 identifiziert werden kann als Hybridisierungssonde eingesetzt.
   Die Genomfragmente, die nachgewiesenermaßen Teile oder bevorzugt das gesamte VEGF-Gen und die flankierenden Genomabschnitte enthalten, werden isoliert und kloniert. Die elektrophoretische Isolierung des oder der entsprechenden Genomfragmente erfolgt beispielsweise über Elektroelution oder mittels "Low-Melting-Agarose"-Verfahren aus dem entsprechenden Gelbereich.

Zur Klonierung des VEGF-Gens werden die oben hergestellten Genom-Fragmente in bakterielle oder eukaryontische Vektoren inseriert. Besonders bevorzugt werden zunächst bakterielle Plasmid- oder Phagen-Vektoren. Zur Insertion des Genomfragmentes werden doppelsträngige Plasmid- oder Phagenvektor-DNA-Moleküle mit Restriktionsenzymen behandelt, so daß für die Insertion geeignete Enden entstehen.

Als Plasmide dienen bekannte Plasmide, wie z.B. pBR322 und seine Abkömmlinge, z.B. pSPT18/19, pAT153, pACYC184, pUC18/19, pSP64/65.

Als Phagenvektoren dienen z.B. die bekannten Varianten des Phagen Lambda wie z.B. -ZAP, -gt 10/11 oder Phage M13mp18/19.

Die einsetzbaren Restriktionsenzyme sind bekannt z.B. aus Gene Band 92 (1989) Elsevier Science Publishers BV Amsterdam.

Das mit Restriktionsenzym behandelte Plasmid oder der Phagenvektor wird mit einem Überschuß des zu inserierenden DNA-Fragments, z.B. etwa im Verhältnis 5:1, gemischt und mittels DNA-Ligasen behandelt, in den Vektor ligiert. Der Ligationsansatz wird zur Vermehrung der Plasmide oder Phagen in pro- oder eukaryontischen Zellen, bevorzugt in Bakterien (z.B. Escherichia coli Stamm K-12 und seine Derivate) eingebracht und diese vermehrt.

Transformation und Selektion der Bakterien erfolgt wie in Molecular Cloning loc. sit. beschrieben.

Die Identität der Fremd-DNA wird bevorzugt über Hybridierungsexperimente und besonders bevorzugt über Sequenzanalysen verifiziert. Gegebenenfalls werden Subklonierungen vorgenommen.

### 2.2.2 Klonierung des Proteinkinase-Gens

Das Gen, das für die Proteinkinase kodiert, wird auf dem Genom des PPV lokalisiert und anschließend in Teilen oder ganz mit seinen flankierenden Genomabschnitten isoliert.

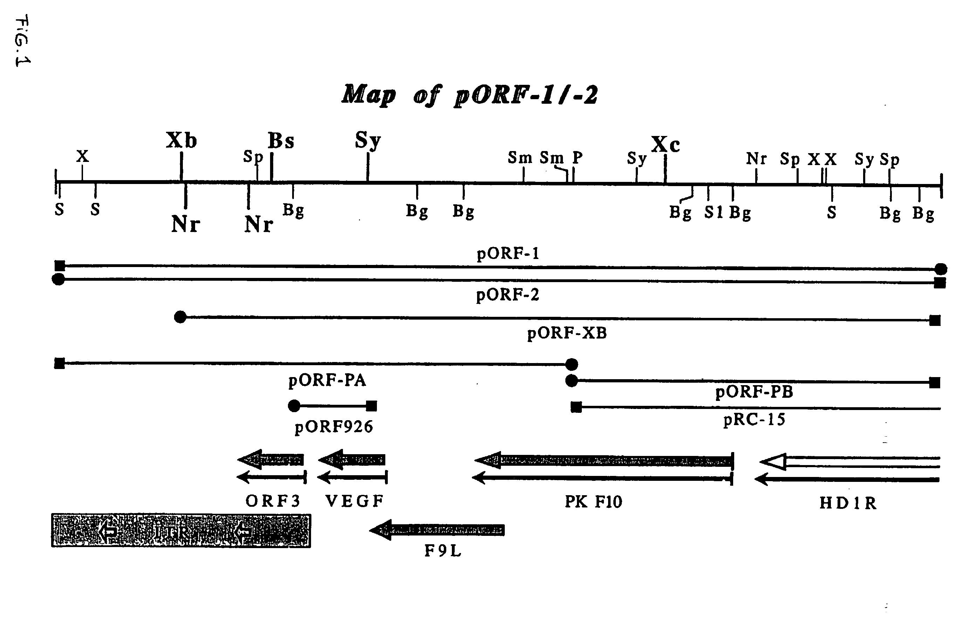

Dieser Bereich kann, wie für die Klonierung des VEGF-Gens beschrieben. über die Fragmentierung des PPV-Genoms (Spaltstellen siehe Abb. 1). bevorzugt mit anschließender Klonierung der Fragmente, und Selektion der Fragmente bzw. Klone, die Teile des PK-Gens oder bevorzugt das gesamte PK-Gen mit flankierenden DNA-Sequenzen des PPV-Genoms enthalten, gewonnen werden. DNA-Moleküle, die als Primer einer PCR oder als Sonden für eine Hybridisierung eingesetzt werden können, lassen sich aus der Sequenz aus Protokoll ID No: 2 bzw. ID No: 9 ableiten.

Gegebenenfalls werden Subklonierungen vorgenommen.

### 2.2.3 Klonierung des Gens, das für das 10kDa-Protein kodiert

Das Gen, das für das 10kDa-Protein kodiert wird entsprechend dem Verfahren, wie es für das VEGF-Gen und das PK-Gen ausführlich beschrieben wurde (oben), auf dem Genom des PPV lokalisiert und in Teilen oder bevorzugt ganz mit seinen flankierenden PPV-Genomsequenzen isoliert.

Dabei wird der Bereich des PPV-Genoms, der das Gen für das 10kDa-Protein oder Teile davon enthält, bevorzugt über PCR und/oder Klonierung und Identifizierung und Selektion der geeigneten Klone erhalten.

DNA-Moleküle, die als Primer einer PCR oder als Sonden für eine Hybridisierung eingesetzt werden können, können Lit. #8 entnommen werden. Gegebenenfalls werden Subklonierungen vorgenommen.

### 2.2.4 Klonierung, des Inverted-Terminal-Repeat-Bereiches bzw. des Genomabschnittes, der zwischen dem PK-Gen und dem HD1R-Gen liegt

Die Vorgehensweise entspricht der ausführlich beschriebenen Klonierung des VEGF-Gens (oben). Die DNA-Moleküle, die zur Isolierung der entsprechenden Bereiche als Primer einer PCR oder als Sonden für eine Hybridisierung eingesetzt werden können, sind im Sequenz Protokoll ID No: 4 (ITR-Region) und No: 7 (Bereich zwischen PK-Gen und HDIR-Gen) ersichtlich.

### 3. Konstruktion von Insertionsplasmiden oder von Deletionsplasmiden.

Auf Basis der gemäß Abschnitt 2 identifzierten, lokalisierten und klonierten Genomfragmente von PPV werden sogenannte Insertionsplasmide hergestellt, die zur Insertion von Fremd-DNA in das Genom der PPV benutzt werden können. Die Insertionsplasmide tragen die in das PPV zu inserierende Fremd-DNA, flankiert von Genomabschnitten des PPV. Es gibt verschiedene Möglichkeiten, die Insertionsplasmide herzustellen: Als Beispiele seien hier erwähnt:

### 3.1 Identifizierung oder Herstellung von singulären Restriktionsenzymerkennungsstellen ("unique restriction sites") in den nach 2.1.4 oder 2.2 erhaltenen klonierten Genomfragmenten und Insertion von Fremd-DNA

Nur einmal vorkommende, d.h. singuläre Restriktionsschnittstellen, lassen sich beispielsweise (s. 2.1.3) den ermittelten PPVNukleotidsequenzen identifizieren.

In diese können synthetisch hergestellte Oligonukleotide, die neue singuläre Schnittstellen für Restriktionsenzyme tragen, eingebaut werden.

Die erhaltenen Plasmide werden wie zuvor beschrieben vermehrt und selektiert.

Alternativ können mittels "PCR", wie von Jacobs et al. (Lit. #12) beschrieben, neue singuläre Restriktionsenzymerkennungsstellen in die PPV-Genomfragmente eingebaut werden .

Die identifizierten und/oder hergestellten singulären Restriktionsenzymerkennungsstellen dienen zur Insertion von Fremd-DNA in das PPV-Genom.

Die Insertion der Fremd-DNA erfolgt durch bekannte Methoden (Lit. #11).

### 3.2 Deletion von Genomsequenzen in den klonierten Genomfragmenten und Insertion von Fremd-DNA

Die Deletion von Subfragmenten aus den klonierten PPV-Genomfragmenten kann beispielsweise durch Behandlung mit Restriktionsenzymen erfolgen, die bevorzugt mehr als eine, besonders bevorzugt 2 Erkennungsstellen besitzen. Nach der Enzymbehandlung werden die erhaltenen Fragmente, wie oben beschrieben beispielsweise elektrophoretisch aufgetrennt, isoliert und die entsprechenden Fragmente durch Ligase-Behandlung wieder zusammengefügt. Die erhaltenen Plasmide werden vermehrt und die deletierten Plasmide selektiert.

Alternativ wird eine singuläre Restriktionsenzymerkennungsstelle auf dem PPV-Genomfragment als Startpunkt für einen bidirektionalen Abbau der Fragmente mit einer Endonuklease, beispielsweise dem Enzym Bal31, genutzt. Die Größe der Deletion kann durch die Dauer der Einwirkung des Enzyms bestimmt werden und gelelektrophoretisch überprüft werden. An die neu entstandenen Fragmentenden werden synthetische Oligonukleotide, wie unter 3.1 (oben) beschrieben, ligiert werden.

Die Fremdgenübertragung in das PPV-Genom gelingt nur bei einem geringen Prozentsatz der Gesamt PPV-Population.

Deshalb sind zur Trennung rekombinanter vom Wildtyp-PPV Selektionssysteme nötig (Lit. #Moss).

Bevorzugt wird das gpt-Selektionssystem eingesetzt, welches auf dem Guanyl-Phosphoribosyl-Transferasegen von E.coli basiert. Dieses Gen vermittelt, exprimiert in einer eukaryotischen Zelle, Resistenz gegen Mycophenolsäure, einen Inhibitor des Purin-Metabolismus. Sein Einsatz bei der Konstruktion rekombinanter Vektor-Viren ist mehrfach beschrieben (s. Lit. #16 / #17).

### 4. Konstruktion eines rekombinanten PPV gemäß 1 bis 12.

Die Insertion von Fremd-DNA in das PPV-Genom erfolgt durch:
a. Gleichzeitige Transfektion der Insertions- oder Deletionsplasmid-DNA und Infektion mit PPV in geeigneten Wirtszellen,
b. Transfektion der Insertions- oder Deletionsplasmid-*DNA* und anschließende Infektion mit dem PPV in geeigneten Wirtszellen,
c. Infektion mit dem PPV und anschließende Transfektion mit der Insertions-oder Deletionsplasmid-DNA in geeigneten Wirtszellen.

Die Methoden der hierzu geeigneten Verfahren sind bekannt. Die Transfektion kann mittels bekannter Methoden erfolgen wie z.B. Calcium-Phosphat-Technik, Liposomen-vermittelte Transfektion oder Elektroporation (s. Lit. #18).
1. Infektion mit PPV:
   Für die Herstellung der PPV mit Fremd-DNA werden Zellkulturen bevorzugt, die eine gute Virusvermehrung und eine effiziente Transfektion erlauben, beispielsweise die permanente Rindernierenzelle BK-K1-3A.
2. Herstellung der Insertions- oder Deletionsplasmid-DNA:
   Die nach den vorher beschriebenen Verfahren erhaltenen transformierten Zellen, beispielsweise Bakterien, die Insertions-oder Deletionsplasmide enthalten, werden vermehrt und die Plasmide in an sich bekannter Weise aus den Zellen isoliert und weiter gereinigt. Die Reinigung erfolgt z.B. durch eine isopyknische Zentrifugation im Dichtegradienten von z.B. CsCl. oder durch Affinitätsreinigung an kommerziell erhältlichen Silikapartikeln.
3. Transfektion:
   Zur Transfektion wird bevorzugt gereinigte Plasmid-DNA zirkular oder linearisiert eingesetzt. Die Reinigung erfolgt z.B. wie unter Abschnitt 2 (oben) angegeben.
4. Kultivierung transfizierter und infizierter Zellen
   Die Zellen werden nach den oben beschriebenen Methoden kultiviert. Beim Auftreten eines zytopathogenen Effektes wird das Kulturmedium entfernt, gegebenenfalls durch Zentrifugation oder Filtration von Zelltrümmern befreit und gegebenenfalls gelagert sowie nach den herkömmlichen Methoden der Einzel-Plaque-Reinigung von Viren aufgearbeitet.

Folgendes Verfahren wird bei der Herstellung rekombinanter PPV angewandt:

Konfluent ausgewachsene BK-KL-3A Zellen werden mit einer Infektionsdosis von 0,001 bis 5, bevorzugt 0,1 MOI (multiplicity of infection) infiziert. Zwei Stunden später werden die infizierten Zellen beispielsweise mit DNA (2-10 µg) des Plasmids pMT-10 entweder nach der CaPO₄-Gly-cerolschock-Methode oder unter Verwendung eines Transfektions-Kits nach Angaben des Herstellers (DOSPER, Boehringer-Manaheim) transfiziert. Anschließend werden diese Zellkulturen mit Medium während drei bis sechs Tagen bei 37°C, 5 % CO₂-Atmosphäre inkubiert, bis cpe oder Plaquebildung sichtbar wurde.

Die Identifizierung von rekombinanten PPV erfolgt in Abhängigkeit von der inserierten Fremd-DNA z.B. durch:
a. Nachweis der Fremd-DNA, z.B. durch DNA/DNA-Hybridisierungen
b. Die Amplifikation der Fremd-DNA mittels der PCR
c. Expression der Fremd-DNA mit Hilfe der rekombinanten Viren

### zu a.

Hierzu wird die DNA aus dem fraglichen Virus gewonnen und mit Nukleinsäure, die zumindest in Teilen mit der inserierten Fremd-DNA identisch ist, hybridisiert.

Die Einzel-Plaque-gereinigten und als Rekombinanten identifizierten PPV werden bevorzugt nochmals auf Vorhandensein und/oder Expression der Fremd-DNA geprüft. Rekombinante PPV, die die Fremd-DNA stabil enthalten und/oder exprimieren, stehen für die weitere Anwendung zur Verfügung.

### zu c.

Die Expression der Fremd-DNA auf Protein-Ebene kann nachgewiesen werden durch z.B. Infektion von Zellen mit dem Virus und anschließender Immunfluoreszenzanalyse mit spezifischen Antikörpern gegen das von der Fremd-DNA kodierte Protein oder durch Immunpräzipitation oder Western Blotting mit Antikörpern gegen das von der Fremd-DNA kodierte Protein aus den Lysaten infizierter Zellen.

Die Expression der Fremd-DNA auf RNA-Ebene kann durch Nachweis der spezifischen Transkripte erfolgen. Hierzu wird RNA aus Virus infizierten Zellen isoliert und mit einer DNA-Sonde hybridisiert, die zumindest in Teilen identisch ist mit der inserierten Fremd-DNA.

### Beispiele

Die folgenden Beispiele beschreiben einen Bereich des PPV-Genoms, der sich für die Insertion und Expression homologer und heterologer Gene oder Teilen davon eignet. Geeignete genomische Fragmente sind auf dem HindIII-Fragment I des PPV ovis-Stammes D1701 (und seiner jeweiligen Derivate) enthalten (Sequenz Protokoll ID No: 8 und ID No: 12).

### 1. Klonierung des HindIII-Fragments I:

Nach Spaltung gereinigter viraler DNA mit dem Restriktionsenzym HindIII wurden die resultierenden DNA-Fragmente durch Agarosegel-Elektrophorese getrennt, das etwa 5,6 kbp große Fragment I ausgeschnitten und mittels der Qiaex^{®}-Methode (Qiagen) isoliert und gereinigt. Dieses DNA-Fragment wurde mit Standardtechniken (Maniatis et al.) in das mit HindIII gespaltene und mit CIP (Kälberdarm-Phosphatase) behandelte Vektorplasmid pSPT18 (Boehringer, Mannheim) kloniert. Die resultierenden rekombinanten Plasmide pORF-1 und pORF-2 unterscheiden sich nur durch die Orientierung des Inserts. Die Anfertigung einer Restriktionskarte ermöglichte eine weitere Subklonierung, wie es in Abb. 1 gezeigt wird. Alle rekombinanten Plasmid-DNAs wurden durch Southern-Blot-Hybridisierung auf restriktionsverdaute virale oder Plasmid-DNA getestet, um ihre Identität und virale Herkunft zu überprüfen.

### 2. DNA-Sequenzierung

Die DNA-Sequenzierung wurde durch Verwendung von doppelsträngiger DNA der verschiedenen rekombinanten Plasmide und SP6- sowie T7-spezifischen Primern, die an beide Enden der Klonierungsstelle des Vektorplasmids pSPT18 binden, bewerkstelligt. Das Didesoxy-Kettenterminationsverfahren nach Sanger wurde nach den Empfehlungen des Herstellers (Pharmacia - Biotech) in Gegenwart von ³⁵S-[α]-dATP und T7-DNA-Polymerase durchgeführt. Gemäß der erhaltenen DNA-Sequenz wurde eine Vielzahl von Oligonukleotiden synthetisiert und zum Sequenzieren beider Stränge des HindIII-Fragments I verwendet. Um Sequenzierungsartefakte oder Bandenkompression aufgrund des relativ hohen G+C-Gehalts des viralen DNA-Inserts (64,78 %) aufzulösen, wurde 7-Desaza-GTP verwendet, und die Sequenzierungsprodukte wurden, falls notwendig, in formamidhaltigen denaturierenden Polyacrylamidgelen getrennt.

### 3. Identifizierung potentieller Gene

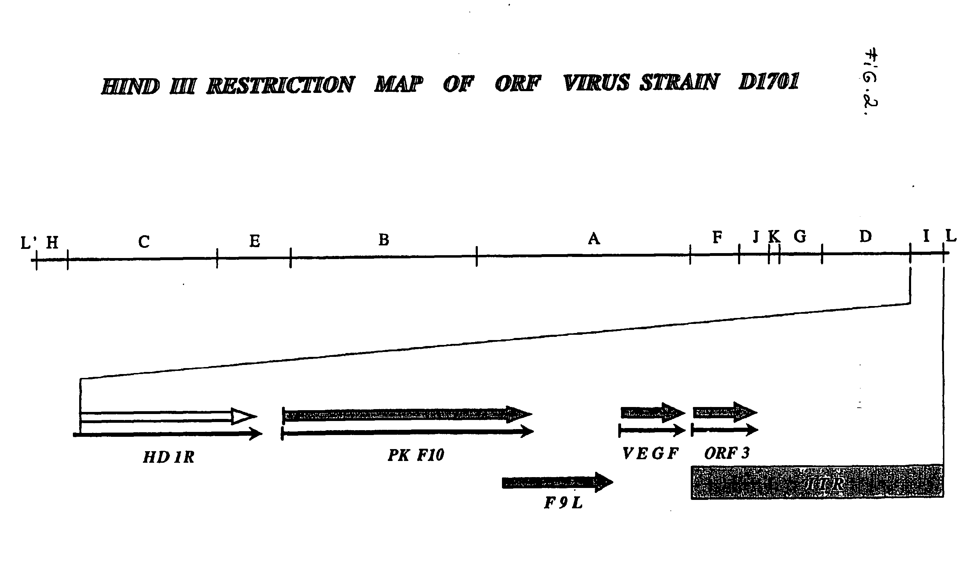

Eine computergestützte Analyse der erhaltenen DNA-Sequenz (Sequenzprotokoll ID No: 8 und ID No: 12) offenbarte mehrere mögliche offene Leserahmen (ORF). Die von diesen ORFs abgeleitete Aminosäuresequenz wurde für Genhomologierecherchen (z.B. Programm GCG) verwendet. Als Ergebnis wurden signifikante Aminosäurehomologien mit den folgenden Genen nachgewiesen (siehe auch Abb. 1).
**3.1** Ein ORF mit Aminosäurehomologie (36,1 bis 38,3 % Identität; 52,8 bis 58,6 % Ähnlichkeit) zum vaskulären endothelialen Wachstumsfaktor (VEGF) verschiedener Säugerspezies (z.B. Maus, Ratte, Meerschweinchen, Rind, Mensch) als auch zu dem VEGF-Gen-Homolog, der vor kurzem in den PPV-Stämmen NZ-2 und NZ-7 beschrieben wurde (Lit. #6), wurde gefunden. Ein entsprechendes Genhomolog ist bei anderen Pockenviren, wie verschiedenen Orthopoxviren, nicht bekannt. Dieser ORF, der als VEGF bezeichnet wird, umfaßt 399 Nucleotide und kodiert ein Polypeptid mit 132 Aminosäuren mit einem berechneten Molekulargewicht von 14,77 kDa. Transkriptionsanalysen unter Verwendung von Northern-Blothybridisierung, RNA-Schutzversuchen und Primerextensionstests mit gesamter oder Oligo (dT) - selektierter RNA, die aus infizierten Zellen isoliert wurde, belegten die Expression von VEGF als frühes Gen ab etwa 2 Stunden nach der Infektion (p.i.). Es wurde gefunden, daß die spezifische mRNA 422 bis 425 Basen abdeckt, beginnend direkt stromabwärts einer Sequenz, die 100 % Homologie mit dem kritischen Bereich eines Consensusmotivs zeigt, das typisch für Promotoren früher Gene des Vaccinia-Virus ist. Das 3'-Ende der VEGF-mRNA konnte in eine Consensussequenz kartiert werden, die frühen Transkripten von z.B. Vaccinia-Virus-Genen gemeinsam ist. Durch Northern-Blothybridisierung wurde die Größe dieser mRNA auf ungefähr 500 Basen geschätzt, was auf einen Poly(A)-Abschnitt mit einer Länge von etwa 100 Basen hinweist.
**3.2** Ein weiterer ORF wurde gefunden, der für eine potentielle Proteinkinase (PK) mit Homologie zu einem entsprechenden Gen kodiert, das in mehreren Orthopoxviren (z.B. Vaccinia-, Variola- oder Shope-Fibroma-Virus) vorkommt und als F10L bekannt ist. Dieses Gen-Homolog wird im Infektionszyklus von PPV-Stamm D1701 spät transkribiert (ab 12 bis 16 Stunden p.i.). Der Transkriptionsstartpunkt befindet sich kurz stromabwärts eines Bereichs, der eine hohe Homologie zu bekannten Promotoren später Gene des Vaccinia-Virus zeigt.
**3.3** Weitere mögliche ORFs wurden gefunden, die bisher keine auffälligen Homologien zu bekannten Gensequenzen zeigen. Von besonderem Interesse ist ein potentielles Gen, das als Gen HD1R bezeichnet wird (Abb. 1). Analysen wie die oben beschriebenen zeigten die Transkription einer spezifischen frühen mRNA mit einer Größe von ca. 1,6 kb.
**3.4** Schließlich wurde mittels Computer ein ORF gefunden, der das 3'-Ende von F10L und das 5'-Ende von VEGF überlappt (F9L, Abb. 1). Der Sequenzvergleich zeigte Homologie mit dem F9L-Gen des Vaccinia-Virus.
**3.5** Verglichen zu bekannten DNA Sequenzen der Orf Stämme NZ-2 und NZ-7 ließ sich der Beginn der sogenannten ITR. Region im Genom von D 1701 bei Nukleotidposition 1611 des HindIII-Fragments I festlegen. Bei der ITR-Region handelt es sich um einen am Ende des Pockenvirusgenoms auftretenden Sequenzbereich, der am anderen Genomende in umgekehrter Orientierung ebenfalls vorliegt und daher als "inverted repeat region" (ITR) bezeichnet wird (siehe Sequenzprotokoll ID No: 4). Der Befund des Sequenzvergleichs deckt sich mit Versuchen zur Genomlokalisation des in pORF-1 klonierten Hindin-Fragments I von D1701. Die Kartierung dieses Fragments und des vermutlich identischen HindIII-Fragments H ist in Abb. 2 dargestellt. Hieraus läßt sich schließen, daß die ITR des D1701 Genoms ca. 2,6 kbp umfaßt.
   Versuche zur Bestimmung des 3' Endes der VEGF mRNA von D1701 enthüllten, daß zwischen ca. 40 und 220 bp nach dem Übergang zur ITR in der ITR mindestens eine weitere virusspezifische RNA startet. Das entsprechende Gen wurde aufgrund der Aminosäurehomologie zu NZ-2 als ORF3 bezeichnet (Abb. 1). Vor dem putativen 5' Ende der ORF3-mRNA befindet sich eine Konsensussequenz, die typisch für einen frühen Promotor von Pockenviren ist. Homologie zu anderen bekannten Genen konnte bisher nicht gefunden werden.

### 4. Einführung von DNA-Sequenzen in das HindIII-Fragment I

Die Möglichkeit der Verwendung des beschriebenen HindIII-DNA-Fragments (in den Plasmiden pORF-1 und pORF-2 kloniert) für die Einführung homologer oder heterologer DNA-Sequenzen wurde erarbeitet. Im folgenden wurden drei verschiedene Strategien angewandt, um dieses Ziel zu erreichen.

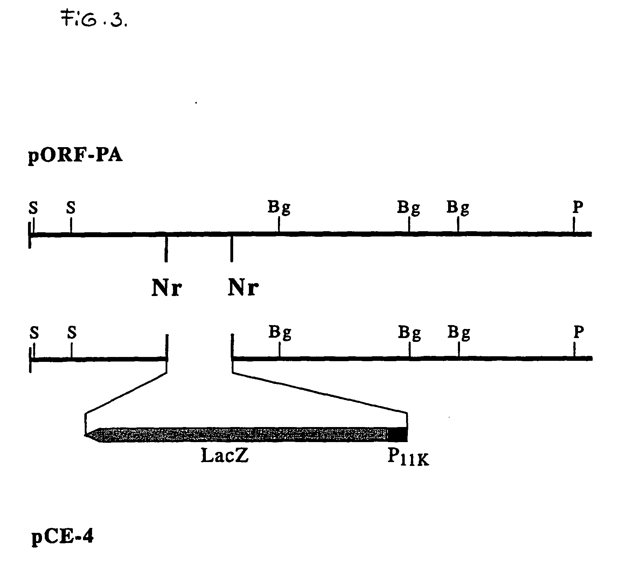

Für die folgenden Beispiele wurde das Plasmid pGSRZ konstruiert, das das funktionelle LacZ-Gen von E. coli unter der Kontrolle des 11K-Promotors des Vaccinia Virus enthält. Zu diesem Zweck wurden die relevanten DNA-Teile des Plasmids pUCIILZ (s. Lit. #7) isoliert und in das Plasmid pSPT18 einkloniert. Diese funktionelle 11K-LacZ-Genkombination (im Folgenden als LacZ-Kassette bezeichnet) kann durch Isolierung eines 3,2-kbp großen SmaI-SalI-Fragments von pGSRZ erhalten werden (Abb. 3).

### Konstruktion der Selektionskassetten

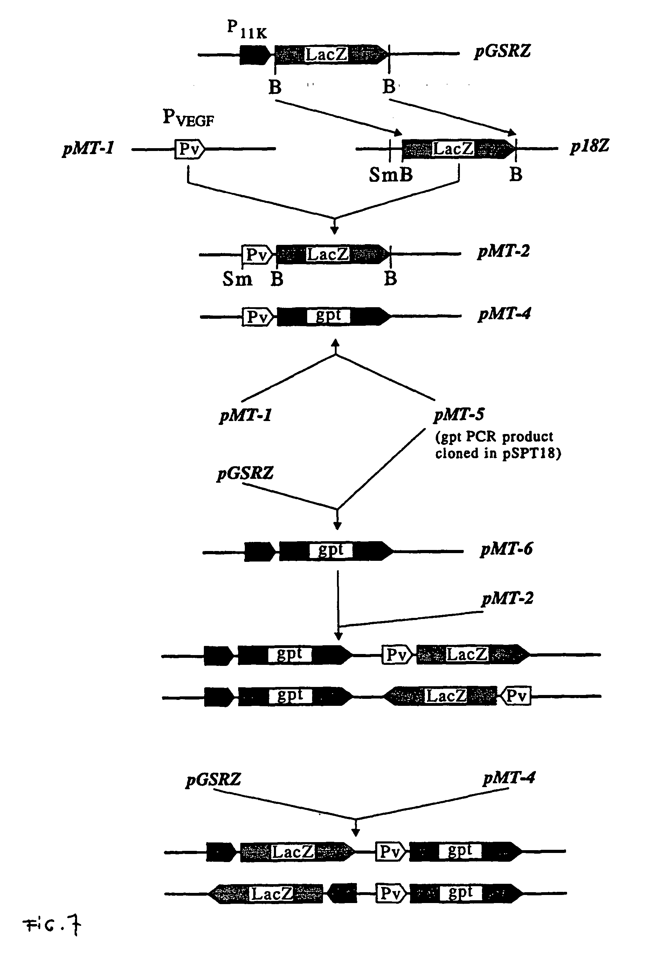

Ausgehend von den Plasmiden pGSRZ (enthält das LacZ Gen unter der Kontrolle des Vaccinia Viruspromotors P_{11K}), pMT-1 (enthält den PPV VEGF-Promotor) und pMT5 (enthält das E. coli gpt-Gen) wurden verschiedene sogenannte Selektionskassetten konstruiert, wie in Abbildung No: 7 dargestellt. Synthetische komplementäre Oligonukleotide, die die Sequenz des VEGF-Promotors (P_{VEGF}) repräsentieren, wurden hergestellt und in die Smal-Schnittstelle von pSPT18 inseriert (pMT-1). Anschließend wurden die Plasmide pMT-2 und pMT-4 hergestellt, indem das LacZ-Gen aus p18Z (erhalten durch Insertion des BamHI-Fragments aus pGSRZ in pSPT18) bzw. das gpt-Gen aus pMT-5 durch BamHI-Spaltung entfernt und in pMT-1 eingefügt wurde (Abb. 7).

Das funktionelle gpt-Gen wurde mittels PCR von dem gpt-Plasmid pMSG (Pharmacia-Biotech) amplifiziert und in den Vektor pCRII durch sogenannte TA-Klonierung entsprechend den Angaben des Herstellers kloniert (Invitrogen Inc.).

Im folgenden konnten, wie in Abb. 7 schematisch dargestellt, Doppel-Selektionskassetten konstruiert werden, welche in den gezeigten Kombinationen und Orientierungen das LacZ-Gen bzw. gpt-Gen unter der Kontrolle des 11K-Promotors und/oder des P_{VEGF} exprimieren.

Entsprechend Beispiel XX (LacZ-VEGF-Deletion) oder YY (intergenisch-Ba131) lassen sich diese Selektionskassetten nach entsprechender Restriktionsenzymspaltung und Isolierung in die verschiedenen PPV orf DNA-Plasmide inserieren. Nach Insertion einer Doppel-Selektionskassette in das beschriebene Plasmid pdV-500, das eine 312 bp große Deletion des VEGF-Gens von PPV D 1701 aufweist, wurde das Plasmid pMT-10 konstruiert. Durch diese Konstruktion wurden die funktionellen LacZ- und gpt-Gene anstelle des durch die Deletion entfernten VEGF-ORF eingesetzt. Nach transienten Expressionstests ließ sich die Aktivität des LacZ-Gens in PPV D1701-infizierten Zellen beweisen (nicht gezeigt), so daß im weiteren eine Selektion auf gpt- und lacZ-exprimierende VEGF-Deletionsmutanten des D1701 durchgeführt wurde.

### 4.1 Insertion in intergenische, nichtcodierende Bereiche

Identifizierung oder Schaffung neuer singulärer Restriktionsstellen, die sich in einem intergenischen, nicht-kodierenden Teil befinden. Diese Stellen werden dann verwendet, um fremde DNA-Sequenzen zu inserieren, die funktionelle und nachweisbare Genprodukte (z.B. das LacZ-Gens, von E. coli) oder Teile davon kodieren.

### Beispiel 4.1.1

Das Plasmid pORF-PB (Abb. 1) enthält eine einzelne NruI-Schnittstelle, die zwischen der Proteinkinase (F10L) und dem HD1R-Gen liegt. Nach Linearisierung von pORF-PB mit diesem Restriktionsenzym wurde die LacZ-Kassette (nach Auffüllreaktion) 'blunt end' ligiert. Rekombinante Plasmide, die das funktionelle LacZ-Gen enthielten, wurden nach Spaltung z.B. mit BglI selektiert, und die korrekte Insertion durch Southern Blothybridisierung mit einer LacZ-spezifischen Sonde sowie durch partielle DNA-Sequenzierung des Übergangs der LacZ- und PPV-DNA nachgewiesen.

### Beispiel 4.1.2

Die gleiche Vorgehensweise wie im obigen Beispiel beschrieben, wurde stromabwärts des VEGF-Gens (Spaltung an der BstEII-Stelle, Abb. 1) und im potentiellen Gen ORF 3 in der ITR-Region verwendet (partieller Abbau mit XbaI, um eine Spaltung der XbaI-Stelle in den pSPT18-Klonierungsstellen zu verhindern).

### Beispiel 4.1.3

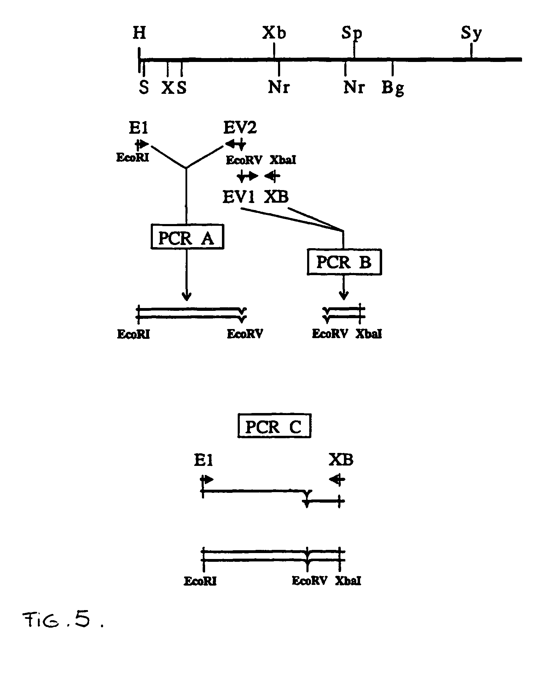

Um eine neue, singuläre Restriktionsstelle an jeder gewünschten Stelle von klonierten PPV DNA-Fragmenten einzuführen, kann die Technik der PCR-Mutagenese verwendet werden (siehe Abb. 10, Lit. #12). Zu diesem Zweck werden zwei PCR-Reaktionen getrennt durchgeführt, wobei das Primerpaar E1+EV2 (PCR A) bzw. EV1+XB (PCR B) verwendet wird. Alle Primer decken 25 Nukleotide ab, die an den angegebenen Sequenzpositionen mit der PPV-DNA-Sequenz identisch sind. Während Primer XB die authentische Sequenz z.B. um die XbaI-Stelle darstellt (Abb. 1), wurde in Primer E1 am 5'-Ende eine neue EcoRI-Stelle und in die Primer EV1 und EV2 (die komplementär zueinander sind) eine neue EcoRV-Stelle eingeführt. Die EcoRV-Stelle, die in der gesamten Sequenz von pORF-1 oder -2 ursprünglich nicht vorkommt, wurde an die Stelle gebracht, die für die Einführung der LacZ-Cassette gewählt wurde. Die aus den Reaktionen A und B erhaltenen PCR-Produkte werden gereinigt, zu Einzelsträngen denaturiert und unter Reassoziationsbedingungen zusammengemischt; anschließend werden sie für die letzte Reaktion PCR C verwendet. Als Primer werden nun E1 und XB verwendet, um in diesem Beispiel die linken 793 bp von pORF-1 zu verlängern. Nach der Gelisolierung und -reinigung wird das resultierende PCR-Produkt von Reaktion C mit EcoRI und XbaI gespalten und anschließend mit dem durch EcoRI und XbaI gespaltenen Plasmid pORF-XB ligiert. Als Ergebnis enthält das Plasmid pORF-1EV (Abb. 5) die EcoRV-Restriktionsstelle an der gewünschten Position; sie kann dann für eine Linearisierung und Ligation mit der LacZ-Cassette verwendet werden.

Außerdem können für das in diesem Beispiel beschriebene Verfahren Fehlpaarungs-Primer verwendet werden, die definierte Basenänderungen oder eine Deletion einer einzelnen Base enthalten, um z.B. Translations-Stopkodons oder Aminosäuredeletionen an jeder gewünschten Stelle in der viralen DNA-Sequenz zu erzeugen.

### 4.2 Intragenische Insertion ohne Deletion von Orf-Sequenzen

In die kodierenden Sequenzen eines der beschriebenen ORFs können nach Spaltung an Restriktionsstellen, die in dem gewählten Gen nur einmal vorkommen, neue oder zusätzliche Sequenzen eingeführt werden.

### Beispiel 4.2.1

Die singuläre XcmI-Stelle, die sich im rechten Teil des Gen-Homolog F10L befindet, wurde verwendet, um das Plasmid pORF-1 zu linearisieren. Sowohl die LacZ-Kassette als auch die gespaltene pORF-1-DNA wurden durch T4- oder Klenow-DNA-Polymerase mit glatten Enden versehen, ligiert und kompetente E. coli-Bakterien (DHSαF') wurden für die Transformation verwendet. Die resultierenden Bakterienkolonien wurden durch Kolonie-Filterhybridisierung unter Verwendung einer LacZ-spezifischen Sonde auf positive rekombinante Plasmide getestet und anschließend wurden die entsprechenden Plasmid-DNAs einer Restriktionsspaltung unterzogen.

### Beispiel 4.2.2

Der VEGF-kodierende Bereich enthält eine einzelne StyI-Stelle, die wie oben beschrieben verwendet wurde, um die LacZ-Kassette zu inserieren.

### 4.3 Deletion viraler Sequenzen

In den folgenden Beispielen wird die Entfernung sowohl codierender (intragenische Deletionen) als auch nicht-kodierender (intergenische Deletionen) Bereiche beschrieben:

### Beispiel 4.3.1

Durch Verwendung von Restriktionsenzymen werden definierte Teile des HindIII-DNA-Fragments I entfernt, um die deletierten viralen Sequenzen zu ersetzen, indem man fremde Gene oder Teile davon insertiert. Dies erfolgte durch Spaltung des Plasmids pORF-PA mit dem Restriktionsenzym NruI (an einer Stelle im ITR-Bereich, Abb. 1), wobei ein 396-bp-Fragment deletiert wurde. Nach einer Auffüllreaktion wurde die LacZ-Kassette wie oben beschrieben ligiert. Abbildung 4 zeigt schematisch die Deletion des 396-bp-Fragmentes aus pORF-PA und die Insertion der LacZ-Kassette. Die Abbildungen 5 und 6 zeigen die so konstruierte Deletions-/Insertionsplasmide pCE4 und pCE9 abstammend von pORF-PA.

### Beispiel 4.3.2

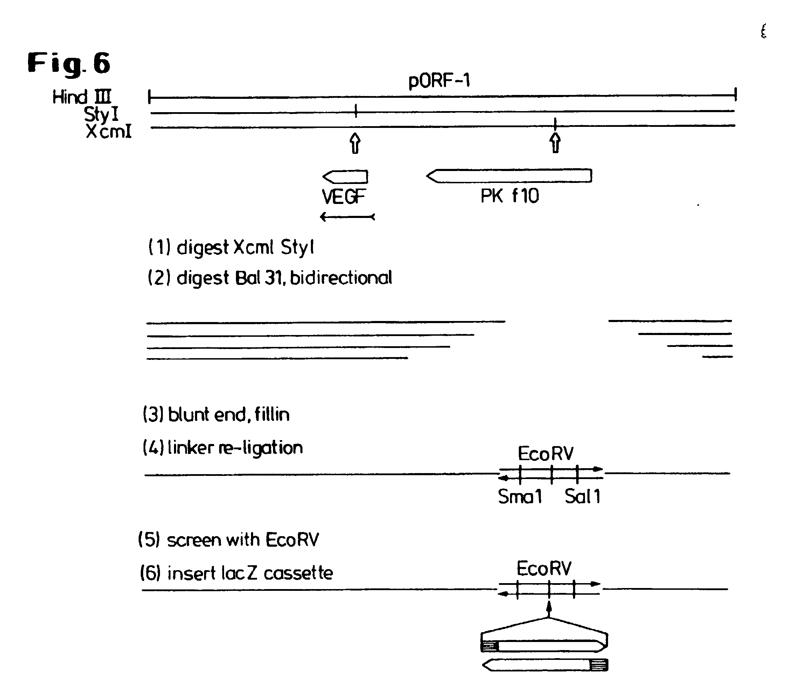

Einzelne Restriktionsstellen im HindIII-DNA-Fragment wurden als Startpunkte verwendet, um eine bidirektionale Deletion von Sequenzen durch Einwirkung der Endonuclease Bal31 zu bewirken, wie es in Abb. 6 schematisch skizziert ist. Zum Restriktionsabbau wurden beim Plasmid pORF-PA das Enzym StyI und beim Plasmid pORF-1 oder pORF-XB das Enzym XcmI verwendet, um die Gene zu öffnen, die für VEGF bzw. Proteinkinase F10L kodieren (Abb. 1 und 6). Nach Zugabe der Exonuclease Ba131 wurden alle 2 Minuten gleiche Teile der Reaktion entnommen und die Reaktion gestoppt. Spaltung der Zeitwerte z.B. mit dem Restriktionsenzym BglI und anschließende Gelelektrophoresen erlaubten die Abschätzung der Größe des durch Bal31 deletierten DNA-Abschnittes. Gemische von den geeigneten Zeitpunkten wurden dann verwendet, um die DNA-Enden durch eine Auffüllreaktion zu schließen. Zwei komplementäre Oligonukleotide, die neue singuläre SmaI-, SalI- und EcoRV-Restriktionsstellen darstellen, wurden hybridisiert und die resultierenden doppelsträngigen Primermoleküle (als "EcoRV"-Linker bezeichnet) an die mit glatten Enden versehenen Ba131-Produkte ligiert. Nach Transformation von Bakterien wurde Plasmid-DNA isoliert und mit EcoRV gespalten, das in der DNA-Sequenz des PPV-HindIII-Fragments I keine Erkennungsstelle enthält. Daher enthielt jede Plasmid-DNA mit einer EcoRV-Stelle die inserierte Linkersequenz und wurde dann für die Ligation der mit glatten Enden versehenen LacZ-Kassette in die neue EcoRV-Stelle verwendet. Die genaue Größe der erzeugten DNA-Deletion in jeder resultierenden rekombinanten Plasmid-DNA konnte durch Sequenzieren mit den einzelsträngigen EcoRV-Linkern sowie mit geeigneten LacZ-genspezifischen Primern bestimmt werden.

### 5. Nachweis und Identifizierung des VEGF-Gens in anderen Parapoxviren

Die Kenntnis des DNA-Bereichs, der in D1701 das VEGF kodiert, erlaubt die Herstellung spezifischer DNA-Sonden und PCR-Primer, um dieses Gen in anderen Parapoxvirus-Stämmen zu identifizieren, die äußerst unterschiedliche Restriktionsprofile haben können. Zu diesem Zweck wurden die folgenden Möglichkeiten getestet: (i) Isolierung eines TaqI-Subfragments (366 bp groß) von pORF-PA als Hybridisierungssonde, das den Mittelteil des VEGF-Gens von D1701 darstellt; (ii) Amplifikation des vollständigen VEGF-ORF mit Hilfe geeigneter synthetischer Primer und anschließendes Plasmidklonieren des PCR-Produkts; (iii) Verwendung von Primern, die verschiedene Teile des VEGF-Gens abdecken, wurden für PCRs in Gegenwart von PPV-DNAs als Matrizen sowie als spezifische Hybridisierungssonden verwendet.

Nach radioaktiver Markierung wurden diese Sonden erfolgreich für Southernsowie Dot/Spot-Blothybridisierung mit der genomischen DNA verschiedener Isolate und Stämme von Parapox ovis, Parapox bovis 1 (Bovines papulöse Stomatitis; BPS) und Parapox bovis 2 (Melker-Knoten) verwendet. Die Southern Blothybridiserung zeigte VEGF-positive Signale mit definierten PPV-DNA Fragmenten, die eine weitere detaillierte Kartierung des VEGF-Gens der verschiedenen PPV erlaubt.

Außerdem können dieselben Sonden für vergleichende RNA-Analysen, wie Northern Blothybridisierung, verwendet werden, um die Expression potentieller VEGF-Gene in anderen PPV-Stämmen zu testen.

### 6. Erzeugung von D1701-Rekombinanten

Konfluent ausgewachsene BK-KL-3A Zellen wurden mit einer Infektionsdosis von 0,1 moi (multiplicity of infection) infiziert. Zwei Stunden später wurden die infizierten Zellen beispielsweise mit DNA (2 bis 10 µg) des Plasmids pMT-10 entweder nach der CaPO₄-Glycerolschock-Methode oder unter Verwendung eines Transfektions-Kits nach Angaben des Herstellers (DOSPER, Boehringer-Mannheim) transfiziert. Anschließend wurden diese Zellkulturen mit Selektivmedium (HAT-Medium +MPA Mycophenolsäure - Xanthin - 5 % FKS) während drei bis sechs Tagen bei 37°C, 5 % CO₂-Atmosphäre inkubiert, bis cpe oder Plaquebildung sichtbar wurde. Je nach Ausprägung des virus-bedingten cpe wurde:
(a) Das Zell-Lysat gewonnen, eine Verdünnungsreihe hergestellt und ein Plaque-Test auf BK-KL-3A Zellen durchgeführt. Das zugegebene Agarose-Mediumgemisch enthielt 0,3 mg/ml Bluo-Gal (Life Science GIBCO-BRL), um blaue Plaques zu identifizieren, die LacZ-exprimierende, MPAresistente D1701-Rekombinanten enthielten.
(b) Nach Bildung von Einzelplaques wurde das in (a) beschriebene Agarose-Bluo Gal-Gemisch zugegeben und blau gefärbte Einzelplaques gepickt.

Die in (a) oder (b) erhaltenen Viren werden - wie oben beschrieben - zur Infektion von BK-KL-3A eingesetzt und mindestens zwei weiteren Plaquetitrationen und -reinigungen unterworfen, bis eine 100 %ige homogene rekombinante Viruspopulation vorliegt.

### 7. VEGF-Promotor

Wie in Kapitel 3.1 skizziert, stellt das VEGF-Gen von D1701 ein frühes Gen dar, die spezifische mRNA wird in D1701 virusinfizierten Zellen von zwei bis vier Stunden nach der Infektion an bis zu späteren Zeiten der Infektion in großen Mengen transkribiert. Daher sollte der identifizierte Promotorbereich des D1701-VEGF sehr nützlich für die Steuerung der Expression fremder Gene oder Teile davon in rekombinanten PPV-Viren sein. Die Sequenz, die den VEGF-Promotor umfaßt (35 bis 40 Nucleotide; Sequenz Protokoll ID No: 6), kann isoliert werden durch (i) PCR unter Verwendung der entsprechenden Primer, die den Promotorbereich flankieren, (ii) Subklonieren des entsprechenden DNA-Fragments oder (iii) Synthetisieren der Promotorsequenz als Oligonukleotid. Nach der Verknüpfung des VEGF-Promotors mit jedem interessierenden Gen oder einer DNA-Sequenz kann die resultierende Genkassette zur Herstellung von rekombinantem PPV nach irgendeinem Verfahren, wie es in den vorangehenden Kapitels beschrieben ist, verwendet werden.

### 8. 10kDa-Gen

Basierend auf der publizierten DNA-Sequenz des 10 kDa Gens des PPV-Stammes NZ-2 (Lit. #8) wurde eine spezifische PCR zum Nachweis des 10kDa-Gens von PPV etabliert. Nach Durchführung der PCR mit den synthetisch hergestellten Primern 10K-up (5-CAATATGGATGAAAATGACGG-3) und 10k-low (5-CAGACGGCAACACAGCG-3) gelang die Amplifikation eines spezifischen, 297 Basenpaar großen Produktes. Die anschließende Klonierung (TA cloning kit, Invitrogen Inc.) ergab das Plasmid pJS-1, welches das 297 bp PCR-Produkt als EcoRI-Fragment enthielt. Die DNA-Sequenzierung beider DNA-Stränge der Insertion von pJS-1 bewies die Anwesenheit der 10kDa-spezifischen Seqzenz. Danach kodiert D1701 für ein 91 Aminosäuren großes 10kDa Protein, das zu dem des PPV-Stammes NZ-2 93,3 % identische bzw. 96,7 % ähnliche Aminosäuren aufweist.

Die Lokalisierung des 10kDa-Gens im EcoRI-Fragment E (4,25 kbp) des D1701-Genoms erfolgte durch Southern-Blot-Hybridisierung mit radioaktiv markiertem pJS-1. Demnach ist dieses Gen, ähnlich wie bei NZ-2, im rechten Teil des Virusgenoms lokalisiert und beinhaltet die Schnittstelle der HindIII-Fragmente K und G (Abb. 2).

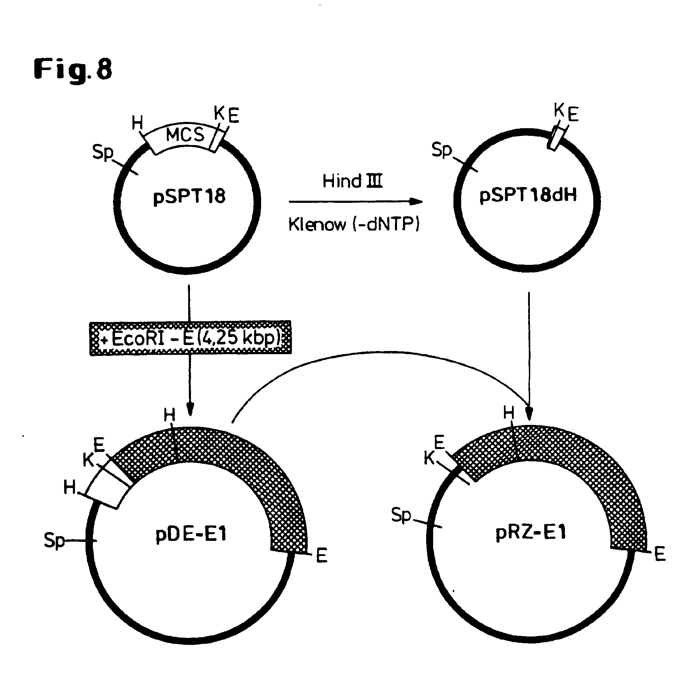

Die Plasmide pDE-E1 und pRZ-E1 die das 4,25 kbp große EcoRI-Fragment E von D1701 enthalten, dienen zur Herstellung von Insertions- und Deletionsplasmiden im 10kDa-Gen (prinzipiell wie vorher beschrieben). Die HindIII-Schnittstelle (Fragmente K-G) im N-terminalen Abschnitt des 10kDa Gens des D1701 (#124-#129 Sequenz ID No: 11) läßt sich sowohl zur direkten Insertion von Fremd-DNA als auch zur Deletion (siehe Ba131 bidirektionaler Verdau) des 10 kDa Gens nutzen. Für letztere Konstruktion wurde die zweite HindIII-Schnittstelle (der multiplen Klonierungsstelle des Vektorplasmids pSPT18) im Plasmid pDE-E1 entfernt. Zu diesem Zweck wurde pSPT18-DNA mit HindIII gespalten, die HindIII-Schnittstelle durch Klenow Behandlung zerstört und religiert. In die EcoRI-Schnittstelle dieses neuen Vektorplasmids pSPT18dH wurde anschließend das 4,25 kbp große EcoRI-Fragment E von D1701 kloniert. Das resultierende Plasmid pRZ-E1 (Abb. 8) besitzt nun eine singuläre HindIII-Schnittstelle im 10 kDa-Gen, welche weitere einfache Manipulation erlaubt.

Southern Blothybridisierung mit pDE-E1 und pJS-1 als radioaktiv markierte Sonden sowie PCR-Untersuchungen zeigen, daß Genome verschiedener PPV bovis 1 keine 10kDa-spezifischen Sequenzen enthalten. Dies weist darauf hin, daß das 10kDa-Gen von PPV nicht essentiell ist (Büttner M. et al. 1996, Lit. #10).

### Literaturverzeichnis

1. Robinson, A.J. and Lyttle, D.J. 1992.
   Parapoxviruses: Their biology and potential as recombinant vaccines. In: Recombinant poxviruses edts. M.M. Binns and G.L. Smith, CRC Press Inc.
2. Mayr, A. 1990.
   Chapter 7 (Ecthyma (Orf) Virus) in: Virus Infections of Vertebrates, Vol. 3: Virus Infections of Ruminants, 1990, Elsevier Science Publishers B.V., The Netherlands.
3. Mazur, C., Rangel Filho, F.B. and Galler, R 1991.
   Molecular analysis of contagious pustular dermatitis virus: A simplified method for viral DNA extraction form scrab material. J.Virol. Methods 35, 265-272.
4. Mercer, A.A.1994.
   10th Int. Conf. on Poxviruses and Iridoviruses 30. April - 5. May 1994. Proceedings.
5. Fleming, S.B., Lyttle, D.J., Sullivan, J.T., Mercer, A.A. and Robinson, A.J. 1995.
   Genomic analysis of a transposition-deletion variant of orf virus reveals a 3.3 kbp region of non-essential DNA. J. gen. Virol. 76, 2669-2678.
6. Lyttle, D.J., Fraser, K.M., Fleming, S.B., Mercer, A.A. and Robinson, A.J. 1994.
   Homologs of vascular endothedial growth factor are encoded by the poxvirus orf virus. J. Virol. 72, 1171-1181.
7. Sutter, G. and Moss, B. 1992.
   Nonreplicating vaccinia vector efficiently expresses recombinant genes. Proc. Natl. Acad. Sci. USA 89, 10847-10851.
8. Naase, M., Nicholson, B.H., Fraser, K.M., Mercer, A.A. and Robinson, A.J. 1991.
   An orf virus sequence showing homology to the 14K fusion protein of vaccinia virus. J.gen. Virol. 72, 1177-1181.
9. Graham, F.L. and van der Eb, A.J. 1973.
   A new technique for the assay of infectivity of human adenovirus 5 DNA. Virology 52, 456-467.
10. Büttner, M., McInnes, C., von Einem, C., Rziha, H.J. and Haig, D. 1996.
   Molecular discrimination of parapoxviruses from different species. 11th Poxvirus and Iridovirus meeting, 4.-9. May, Toledo, Spain.
11. Sambrock, J., Frisch, E.F. and Maniatis, T. 1989.
   Molecular Cloning, A laboratory manual, 2nd edition. Cold Spring Harbor Laboratory Press.
12. Jacobs, L., Rziha, H.-J., Kimman, T.G., Gielkens, A.L.J. and von Oirschot, J.T. 1993.
   Deleting valine-125 and cystein-126 in glycoprotein gl of pseudorabies virus strain NIA-3 decreases plaque size and reduces virulence for mice. Arch. Virol. 131, 251-264.
13. Virologische Arbeitsmethoden, 1989.
   Biochemische und Biophysikalische Methoden, VEB Fischer Verlag.
14. Sharp, P.A., Berk. A.J. and Berger, S.M. 1980.
   Transcription maps of adenovirus. Meth. Enzymol. 65, 750-768.
15. Watson, J.D., Hopkins, N.H., Roberts, J.W., Seitz, J.A. and Weiner, A.M. 1987.
   Molecular Biology of the Gene, Benjamin/Cummins Publishing Company, Menlo Park.
16. Falkner, F.G. and Moss, B. 1988.
   Escherichia coli gpt gene provides dominant selection for vaccinia virus open reading frame expression vectors. J. Virol. 62, 1849-1854.
17. Boyle, D.B. and Coupar, B.E. 1988.
   Construction of recombinant fowlpox viruses as vectors for poultry vaccines. Virus Res. 10, 343-356.
18. Methods in Virology, Vol. VI, 1977.
   edts. Maramorosch, K. and Koprowski H. Academic Press. New York, San Francisco.
19. Hattori, M. and Sakaki Y. 1986.
   Dideoxy sequencing method using denaturated plasmid templates. Anal. Biochem. 152, 232-238.
20. Chomczynski, P. and Sacchi, N. 1987.
   Single step method of RNA isolation by acid guanidinium-thiocyanatephenol-chloroform extraction. Ana. Biochem. 162, 156-159.

### ID No: 1

Die Sequenz ID No: 1 der Anmeldung zeigt das auf dem HindIII-Fragment I des Stammes PPV D 1701 lokalisierte VEGF-Gen.

### Zusätzliche Informationen:

| | |
|---|---|
| Earyl promotor: | Nukleotide 50 bis 64 |
| mRNA-Start: | Nukleotide 78 bzw. 80 |
| mRNA-Stop: | Nukleotide 498 bis 500 |
| Translationsstart: | Nukleotide 92 bis 94 |
| Translationsstop: | Nukleotide 488 bis 490 |

### ID No: 2

Die Sequenz ID No: 2 der Anmeldung zeigt das auf dem HindIII-Fragment I des Stammes PPV D1701 lokalisierte Proteinkinase-Gen F10L (Version 1)

### Zusätzliche Informationen:

| | |
|---|---|
| Late Promotor: | Nukleotide 48 bis 66 |
| mRNA-Start: | Nukleotide 74 bis 78 |
| Translationsstart: | Nukleotide 94 bis 96 |
| Translationsstop: | Nukleotide 1738 bis 1740 |

### ID No: 3

Die Sequenz ID No: 3 der Anmeldung zeigt das auf dem Hindin-Fragment I des Stammes PPV D1701 lokalisierte HD1R-Gessegment.

### ID No: 4

Die Sequenz ID No: 4 der Anmeldung zeigt die auf dem HindIII-Fragment I des Stammes PPV D1701 lokalisierte ITR-Region und das in dieser Region befindliche ORF3-Gen.

### Zusätzliche Informationen:

| | |
|---|---|
| Beginn ITR-Region: | Nukleotid 7 |
| Early Promotor: | Nukleotide 18 bis 33 |
| ORF3 mRNA Start: | Nukleotide 40 bis 41 |
| ORF3 mRNA Stop: | Nukleotide 673 bis 679 |
| ORF3 Translationsstart: | Nukleotide 111 bis 113 |
| ORF3 Translationsstop: | Nukleotide 562 bis 564 |

### ID No: 5

Die Sequenz ID No: 5 der Anmeldung zeigt das auf dem HindIII-Fragment I des Stammes PPV D1701 lokalisierte F9L-Genhomolog (Version 1).

### Zusätzliche Informationen:

| | |
|---|---|
| Startcodon: | Nukleotide 48 bis 50 |
| Stopcodon: | Nukleotide 861 bis 863 |

### ID No: 6

Die Sequenz ID No: 6 der Anmeldung zeigt die auf dem HindIII-Fragment I des Stammes PPV D1701 lokalisierte VEGF-Promotor-Region.

### ID No: 7

Die Sequenz ID No: 7 der Anmeldung zeigt die auf dem HindIII-Fragment I des Stammes PPV D1701 lokalisierte, zwischen dem HD1R- und dem PKF10L-Gen gelegene intergenische Region.

| | |
|---|---|
| Putativer Translationsstop HD1R: | Nukleotide 25 bis 27 |
| Translationsstart PKF10L: | Nukleotide 223 bis 225 |

### ID No: 8

Die Sequenz ID No: 8 der Anmeldung zeigt die vollständige Nukleotidsequenz des HindIII-Fragment I (Version 1) des PPV-Stammes D1701.

### ID No: 9

Die Sequenz ID No: 9 der Anmeldung zeigt Version 2 des auf dem HindIII-Fragment I des Stammes PPV D1701 lokalisierten Proteinkinase F10L-Gens.

### Zusätzliche Informationen:

| | |
|---|---|
| Late Promotor: | Nukleotide 48 bis 66 |
| RNA-Startsignal: | Nukleotide 72 bis 80 |
| mRNA Start: | Nukleotide 74 bis 78 |
| Translationsstart: | Nukleotide 94 bis 96 |
| Translationsstop: | Nukleotide 1585 bis 1588 |

### ID No: 10

Die Sequenz ID No: 10 der Anmeldung zeigt Version 2 des auf dem HindIII-Fragment I des Stammes PPV D1701 lokalisierten F9L-Genhomologs.

### Zusätzliche Informationen:

| | |
|---|---|
| Translationsstart: | Nukleotide 50 bis 52 |
| Translationsstop: | Nukleotide 722 bis 724 |

### ID No: 11

Die Sequenz ID No: 11 der Anmeldung zeigt das auf den EcoRI-Fragment E des Stammes PPV D1701 lokalisierte 10 kDa-Gen.

### Zusätzliche Informationen:

| | |
|---|---|
| Translationsstart: | Nukleotide 5 bis 7 |
| Translationsstop: | Nukleotide 275 bis 277 |

### ID No: 12

Die Sequenz ID No: 12 der Anmeldung zeigt die vollständige Nukleotidsequenz der Version 2 des HindIII-Fragment I des PPV-Stammes D1741.

### ID No:13

Die Sequenz ID No: 13 der Anmeldung zeigt Version 3 des auf dem HindIII-Fragment I des Stammes PPV D1701 lokalisierten Proteinkinase FIOL-Gens.

### Zusätzliche Informationen:

| | |
|---|---|
| Late Promotor: | Nukleotide 48 bis 66 |
| RNA-Startsignal: | Nukleotide 72 bis 80 |
| mRNA Start: | Nukleotide 74 bis 78 |
| Translationsstart: | Nukleotide 94 bis 96 |
| Translationsstop: | Nukleotide 1585 bis 1588 |

### ID No: 14

Die Sequenz ID No: 14 zeigt die Aminosäuresequenz des PPV D1701 Proteinkinase F10L-Homologs (abgeleitet aus Sequenz ID No: 13).

### ID No: 15

Die Sequenz ID No: 15 zeigt die Aminosäuresequenz des PPV D1701 VEGF-Homologs (abgeleitet aus Sequenz ID No: 1).

### ID No: 16

Die Sequenz ID No: 16 zeigt die Aminosäuresequenz des PPV D1701 F9L-Homologs (abgeleitet aus Sequenz ID No: 10).

### SEQUENCE LISTING

(1) GENERAL INFORMATION:
   (i) APPLICANT:
      (A) NAME: BAYER AG
      (B) STREET: Bayerwerk
      (C) CITY: Leverkusen
      (E) COUNTRY: Germany
      (F) POSTAL CODE (ZIP): D-51368
      (G) TELEPHONE: 0214/3061285
      (H) TELEFAX: 0214/30 3482
   (ii) TITLE OF INVENTION: Parapockenviren die Fremd-DNA enthalten, ihre Herstellung und ihre Verwendung in Impfstoffen
   (iii) NUMBER OF SEQUENCES: 16
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patent In Release #1.0, Version #1.30 (EPO)
(2) INFORMATION FOR SEQ ID NO: 1:
   (i) SEQUENCE CHARACTERISTICS:
      (A) LENGTH: 540 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Parapox ovis
      (B) STRAIN: D1701-VEGF-Gen
   (xi) SEQUENCE DESCRIPTION: SEQ ID NO: 1:
   GGTGCGCTAC CAATTCGCGC GGCCGGCCGC GCTGCGCGCG TAGCCGCGCA AAATGTAAAT    60
   TATAACGCCC AACTTTTAAG GGTGAGGCGC CATGAAGTTT CTCGTCGGCA TACTGGTAGC   120
   TGTGTGCTTG CACCAGTATC TGCTGAACGC GGACAGCACG AAAACATGGT CCGAAGTGTT   180
   TGAAAACAGC GGGTGCAAGC CAAGGCCGAT GGTCTTTCGA GTACACGACG AGCACCCGGA   240
   GCTAACTTCT CAGCGGTTCA ACCCGCCGTG TGTCACGTTG ATGCGATGCG GCGGGTGCTG   300
   CAACGACGAG AGCTTAGAAT GCGTCCCCAC GGAAGAGGCA AACGTAACGA TGCAACTCAT   360
   GGGAGCGTCG GTCTCCGGTG GTAACGGGAT GCAACATCTG AGCTTCGTAG AGCATAAGAA   420
   ATGCGATTGT AAACCACCAC TCACGACCAC GCCACCGACG ACCACAAGGC CGCCCAGAAG   480
   ACGCCGCTAG AACTTTTTAT GGACCGCATA TCCAAACGAT GATGCGATCA GGTCATGCGG   540
(2) INFORMATION FOR SEQ ID NO: 2:
   (i) SEQUENCE CHARACTERISTICS:
      (A) LENGTH: 1740 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Parapox ovis
      (B) STRAIN: D1701- Proteinkinase-Gen(Version 1)
   (xi) SEQUENCE DESCRIPTION: SEQ ID NO: 2:
   CGAGTGACTG CCCATCCCGT TGCTGCGCGA CTCGGGACTG CCCTCTGTTT TTCTTTCCCG     60
   TTTCTTCTTA TTAGGTAGTT GTTGCCCACC TCCATGATCC TCGCACGCGC TGGCGGGCGA    120
   CCTCGCACGC CCGCGGCGGC CGCGGCGGCC GCCGAGGACG GCAAGAACAG TGATCGCCGG    180
   AAGCGCAAGC GCAAGACGCC CAACTGCGAA GACGCCGACA ACTCCGACGA CGAGCTAGCG    240
   CAGACGCCGT GCGACCGCGA GTGGCCGGAC TGTCGCGCGA GCTCGATCAC GAGCTCCGAC    300
   TCGGTCTCTC TCGGCGACGA GATCTACTTG CGGTACGTAG CCTCGCAGGT GGACTTCGCG    360
   CAGACCTGGG CCCCGCCGGT GCGGCTGCTG CGCTTCTTCG GGAACTTCTC GAAGGAAACG    420
   CTCAGCCGCA TGTCGCGGCG CGGGTACGTG AACCGCTCCT ACTTCCAGAT GGCGCACGCG    480
   CGCTTCTCGC CCACCAACGA CGACATGTAC CACATGGCCA CTGGCGGGTA CGGCATCGTG    540
   TTCCGCTTCG ACCGCTACGT GGTCAAGTAC GTCTTCGAGC ACCGCAACGG CATGTCCGAG    600
   ATGGACGCCT CTACGGAGTA CACGGTGCCG CGGTTCCTGC GCAATAACCT CAAGGGCGAC    660
   GAGCGCGAGT TCGTGGTCTG CGCGCTGGCC ATGGGGCTGA ACTACCGGCT GGGCTTCCTG    720
   CACTCGCTGT ACCGGCGCGT GCTGCACACG CTGCTGCTGC TCATGCGCGT GGAGGAAGGC    780
   CAGCGGCCCT CGGTAGAGAT GGCCAAGAAG CCGCTGCTGC GCTGGTTCGA GGCGCGCAAG    840
   GACAGCGAGT CCTTCGTGCG CCTGGTCTCG TACTTCTACC CCTCGGCCGT GCAGAGCAAC    900
   GTGAACCTGA TCAACAACTT CCACCACCTG GTGCACTTCT TTGAGCACGA GAAGCGCGCG    960
   CGGTACGTGT TCGACCGCGG GGCCGTGATC GTGTTCCCTC TGGCGCGCGG GTCCGCGGAC   1020
   TCGATCTCGC CGGAGGCGGC GGCAGCGCTG GGCTTCGCGC CGCACTCGGA GTTCCTCAAG    1080
   TTCGTGTTCC TGCAGATCGC GCTGCTGTAC CTGAAGATAT ACGAGCTCCC GGGCTGCACG    1140
   AACTTCCTGC ACGTGGACCT GAAGCCCGAC AACGTGCTCA TCTTCGACAG CGCGCGCGCT    1200
   CAGCGTGACT GCGGCCGGTG CGACTTTTCG CTTCGAAGAG CCCGTGCGCG CGGCGCTGAA    1260
   CGACTTCGAC TTCGCGCGCG TGGCCACCAT CGAGAACCGC AAGATCGCGG GCAGCGTCCG    1320
   CGTGCCGCAG AACTGGTACT ACGACTTCCA CTTCTTCGCG CACACGCTGC TGCGCGCGTA    1380
   CCCGCACATC GCCGCGGAGG ACCCGGGCTT CCACGCGCTG CTCTCGGAGC TCACGGTCTC    1440
   GTGCTCGCGC GGGACCTGCG ACCGCTTCCG GCTGCGCGTG TCCTCGCCGC ACCCCATCGA    1500
   GCACCTCGCG CGGCTGGTGC GCCGCGACGT CTTCTCCCGC TGGATAAATG CCGCCGCGGA    1560
   CGCCCCCGAC GCCGCACTCT CCTGAGCCCA CGCCCGCGGC GCCGGGCTCG CTGTACGACG    1620
   TCTTCCTCGC GCGCTTCCTG CGCCAGCTGG CCGCGCGCGC GGCGCCGGCC TCGGCCGCCT    1680
   GCGCCGTGCG CGTGGGTGCG GTGCGCGGCC GCCTGCGGAA CTGCGAGCTG GTGGTGCTGA    1740
(2) INFORMATION FOR SEQ ID NO: 3:
   (i) SEQUENCE CHARACTERISTICS:
      (A) LENGTH: 1080 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (vi) ORIGINAL SOURCE:
      (A) ORGANISM: Parapox ovis
      (B) STRAIN: D1701-HD1R-Genregion
   (xi) SEQUENCE DESCRIPTION: SEQ ID NO: 3:
   AAGCTTGTTG CGCGAGTACG TGGTGACCCG CGCCTACTCG GATCAGACCG AGCCGATCAT      60
   GGACTTGCTC ATCGGCATGG GCGCCGACGT GGACATGCAG GTCGGCGTGT GCCGCACGGC     120
   GCTGCACGCC TGCCTTACGG GCTTGAACAC GAACCCGTGC ATGATTCGCG CGCTGCTTCG     180
   GCGCGGCGCC AGCGTGACCG CAAAAGACAC CTACGAGATG ACGCCACTGG CGTGTTGCTG     240
   AAGTCCGCGA GCGCGACGCC GGAGCTCGTG CGCATCCTCG TGGAAGCAGG CTCCGACGTG     300
   AGCGCCACCG ACTTCCGCCT CAACGGCATG CTGCACCAGC ACGCAGTCCA CGCGCCCGCG 360
   CGCGAGCGTC ATGCGCGAGC TCATCCGGCT GGGGTGCAGC CCAGCGGCCA AAAACATGTT 420
   TGGGAACACG CCGATGCACA TGCTGGCCAT GGAAAGCTCC TGCCGCCGCT CGCTGATCCT 480
   CCCGCTGCTG GAGGCAGGGC TTTCCGTGAA CGAGGAGAAC CTGCACTACG GCACCGTGCC 540
   TCTGCACGTG GCCTCGGGGT ACGACAACAC GCAGGGCTGC CTCAAGCTCC TCCGGCAGGG 600
   AGGAGACCCC ACCGTCGTGT CAGCCGCCGG ACGCACACCG ATCTCGAACA TGCTCGTCAA 660
   AGCCAACCAC GTGGCGGTCG CCGGCGCGCT GTCGACGCAC CCGAGCGCGG CAGTGGTCGT 720
   GCAGGCTCTC GAGCAGGCTC TCGAGAACGT GCTGAACGCC GGGCCCAGCG AGGCCTCGCG 780
   GCTCGCCGTG GCCTTTGTGG TGGCGCGCGC CGGCGCATCC GCGCTACCGG AGGCCGTGCG 840
   CCGTCTTCAC GAGGGCTTCG TCGCCGACTG CGAGCGCGAA GTCGCGTTGC TTTCCCGCAG 900
   CATGCTCGGC ACACCGGCCG TGAGCGCGCT GGTCGTGCTG GTCAGCAAGG AGGTCTTTGG 960
   CACTGTTATC TCCTCGCGTG CGCTGCGCGT CGCGCGGGAG GTCCGCGTGT ACGCAAGGCC 1020
   GCTCCGCGAG GCGCTCATAA ATCTGCGCCA CAAATGCCGC TTAGTTTCCA GCCTTAAAAG 1080
(2) INFORMATION FOR SEQ ID NO: 4:
   (i) SEQUENCE CHARACTERISTICS:
      (A) LENGTH: 1616 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Parapox ovis
      (B) STRAIN: D1701- ITR und ORF3 -Gen
   (xi) SEQUENCE DESCRIPTION: SEQ ID NO: 4:
   AAGGAGGCTC CACGGAGCAA AGTGAAAAAG GACCGCCTAG AGTCGAGACC CCTCCCTCCC 60
   GCCTCGGGCA AACCCACAGC CGCCGCAAAC ACCACACCCG CCGACCTACC ATGCACCCCT 120
   CGCCGCGCCG GCTGCTCGGC GCGCTCGCGC TGCTGGCGCT GGGCTTCGCT CGGCGCGCTC 180
   TTCGCCCCGC GGCGCCGCTC GTGCCGGCCG CCTTCCTGGA GGTGGGGCAC GTGCGCGCGA     240
   ACCCGTCCGC CTCGGTGACC TGCCTCACGG TGGGCGGCGA CGGGCGGCAC ATGGCGGCGG     300
   TCGCGCACGG CGGCGGGACG CTCTCGCCGG TGTACCCGCT GGCCGCCGGC ATGCACGCGA     360
   CCTTCTCCTC CGCGCGCAAG GGCGCGCTGC TGCTGAACGT CGCGACCGTG ACTGTGTACG     420
   ACGTGCGCGC GCTCGCCCCC GAGTTCGAGC TCGTCTGCAT CGCGGTGGTC GGCGGCTACA     480
   ACTCGGCCGC GGCCGCCACG CGGCCCGCGG CCGAGTGGCA CCGCCAGCTG GAGCTGCGCC     540
   GCTCGGAGCT GTGACCCCTC CCTCCCCGGT CTCCCTCTGT CTTTGTAATC GGCCTTAGAG     600
   ATTAGACATC ATCCTCCACG CCTCTTTGTC CGCCGCCCTT CTTCGCGGAC GGATGAACCA     660
   ATTAATTAAT TATTTTTGTC GCTCGCCCGC TCACTCCGGC AAGGGAACGA GTGACGTTAA     720
   CTCTCTCACC CTCACGCACA AGAACAAGAA CCGCTCACTC ACCGGGCAAG GGAACACGGT     780
   TAAGGTCAAC TCACTCGCGA GAACAAGTTG ACCCTCACTC TAGAGAACGA GGAACGGGCA     840
   ACAAGCAACC GTCAACTCAC TTACCACGAG AACAAGTTGA CCGCCACTCA AAGGGAACAG     900
   AGAACAGTAA CCGTTCTCGC TCGCTCGGAA CAATAGAACA AGTTAACGTC AACTCGCTCG     960
   CTCGGTGTAA GAGAACAACA GAACAAGCAA CTGTTGACCA CTCAACCCCC GGAGAAGAGA    1020
   ACAAGAGAGC AGTCAACTCA CCCACTCAGT CTTGGATGAG AGGAGGACGA GTTAACGAGT    1080
   ACTCGCACGC AGAGTGAGAG AGTGAGGACA TAATAATAGT TAACGAGTTA ATACTCACTC    1140
   GCTCACTCAG AGTGAGAGAG AACCAGTGAG CGAGTTAACC GCGCACACGA GCGAGAGAAC    1200
   AGTGAACTGC TCGCGCGCTC GCTCGGTAGC AGTCGGCCTT TCTTAAAACG GTTCGTAAAA    1260
   CTTTTCCCGA GACAGTTCAC CCTCCAAAAC TTTTAAAACT AAACTCGGAG GTGGCCTGCC    1320
   CTCCACTCTC CGTAAAACTT TTGTAAAACT GTCGGAGGTC GGTCGACTTC GCAACTCGTC    1380
   CGCGAAAACT TTTCGTGGGC AGTGTCTGCC TCTCTCAGGC TCCTCGCATC ACTTTCGCGG    1440
   AGCCTCGAGG TAGGTCACCT CTCTCCAAAC TTTTGTAAAA ACTTTTTCGC GGAGCCTCTG    1500
   GAGGCCGTCC TCCCTCCAAA ACTTTTCGTA AAATCTCTTC GGAGGCCGTC CTCCCTCCAA    1560
   AACTTTTCGT AAAATCTTTG GGAGGTCGAC CTCCCTCAAA ACTTTTTATA AAGCTT        1616
(2) INFORMATION FOR SEQ ID NO: 5:
   (i) SEQUENCE CHARACTERISTICS:
      (A) LENGTH: 900 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Parapox ovis
      (B) STRAIN: D1701- F9L-Gen (Version 1)
   (xi) SEQUENCE DESCRIPTION: SEQ ID NO: 5:
   GCACCTCGCG CGGCTGGTGC GCCGCGACGT CTTCTCCCGC TGGATAAATG CCGCCGCGGA    60
   CGCCCCCGAC GCCGCACTCT CCTGAGCCCA CGCCCGCGGC GCCGGGCTCG CTGTACGACG   120
   TCTTCCTCGC GCGCTTCCTG CGCCAGCTGG CCGCGCGCGC GGCGCCGGCC TCGGCCGCCT   180
   GCGCCGTGCG CGTGGGTGCG GTGCGCGGCC GCCTGCGGAA CTGCGAGCTG GTGGTGCTGA   240
   ACCGCTGCCA CGCGGACGCT GCCGGCGCGC TCGCGCTGGC CTCCGCGGCG CTGGCGGAAA   300
   CGCTGGCGGA GCTGCCGCGC GCGGACAGGC TCGCCGTCGC GCGCGAGCTG GGCGTGGACC   360
   CAGAGCACCC GGAGCTGACG CCGGACCCCG CCTGCGCGGG CGAGAGGCGC GCTTGCGCAG   420
   AACATCGACA TCCAGACGCT GGACCTGGGC GACTGCGGCG ACCCCAAAGG CCGCCGACTG   480
   CGCGTGGCGC TGGTGAACAG CGGCCACGCG GCCGCAAACT GCGCGCTCGC GCGCGTAGCG   540
   ACCGCGCTGA CGCGCCGCGT GCCCGCAAGC CGGCACGGCC TCGCGGAGGG CGGCACGCCG   600
   CCGTGGACGC TGCTGCTGGC GGTGGCCGCG GTGACGGTGC TCAGCGTGGT GGCGGTTTCG   660
   CTGCTGCGGC GCGCGCTGCG GGTGCGCTAC CAATTCGCGC GGCCGGCCGC GCTGCGCGCG   720
   TAGCCGCGCA AAATGTAAAT TATAACGCCC AACTTTTAAG GGTGAGGCGC CATGAAGTTT   780
   CTCGTCGGCA TACTGGTAGC TGTGTGCTTG CACCAGTATC TGCTGAACGC GGACAGCACG   840
   AAAACATGGT CCGAAGTGTT TGAAAACAGC GGGTGCAAGC CAAGGCCGAT GGTCTTTCGA   900
(2) INFORMATION FOR SEQ ID NO: 6:
   (i) SEQUENCE CHARACTERISTICS:
      (A) LENGTH: 94 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Parapox ovis
      (B) STRAIN: D1701- VEGF-Promotor
   (xi) SEQUENCE DESCRIPTION: SEQ ID NO: 6:
   CCGCGCTGCG CGCGCGTAGC CGCGCAAAAT GTAAATTATA ACGCCCAACT TTTAAGGGTG 60
   AGGCGCCATG AAGTTTCTCG TCGGCATACT GGTA 94
(2) INFORMATION FOR SEQ ID NO: 7:
   (i) SEQUENCE CHARACTERISTICS:
      (A) LENGTH: 250 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Parapox ovis
      (B) STRAIN: D1701- Intergen. Region zwischen HD1R und Proteinkinase Gen
   (xi) SEQUENCE DESCRIPTION: SEQ ID NO: 7:
   CAAATGCCGC TTAGTTTCCA GCCTTAAAAG GCAAGTGGGA CCCTGCTCGC TGCCCGGCGA 60
   ACTGGTGGAG CGCGTGCTCG CGACCGTGCC ACTGGCCGAC TTGCGCCGCT CGTGCAGCCG 120
   CCGCGCGCCC GAGTGACTGC CCATCCCGTT GCTGCGCGAC TCGGGACTGC CCTCTGTTTT 180
   TCTTTCCCGT TTCTTCTTAT TAGGTAGTTG TTGCCCACCT CCATGATCCT CGCACGCGCT 240
   GGCGGGCGAC 250
(2) INFORMATION FOR SEQ ID NO: 8:
   (i) SEQUENCE CHARACTERISTICS:
      (A) LENGTH: 5515 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Parapox ovis
      (B) STRAIN: D1701- HIND III Fragment I (Version 1)
   (xi) SEQUENCE DESCRIPTION: SEQ ID NO: 8:
   AAGCTTGTTG CGCGAGTACG TGGTGACCCG CGCCTACTCG GATCAGACCG AGCCGATCAT     60
   GGACTTGCTC ATCGGCATGG GCGCCGACGT GGACATGCAG GTCGGCGTGT GCCGCACGGC    120
   GCTGCACGCC TGCCTTACGG GCTTGAACAC GAACCCGTGC ATGATTCGCG CGCTGCTTCG    180
   GCGCGGCGCC AGCGTGACCG CAAAAGACAC CTACGAGATG ACGCCACTGG CGTGTTGCTG    240
   AAGTCCGCGA GCGCGACGCC GGAGCTCGTG CGCATCCTCG TGGAAGCAGG CTCCGACGTG    300
   AGCGCCACCG ACTTCCGCCT CAACGGCATG CTGCACCAGC ACGCAGTCCA CGCGCCCGCG    360
   CGCGAGCGTC ATGCGCGAGC TCATCCGGCT GGGGTGCAGC CCAGCGGCCA AAAACATGTT    420
   TGGGAACACG CCGATGCACA TGCTGGCCAT GGAAAGCTCC TGCCGCCGCT CGCTGATCCT    480
   CCCGCTGCTG GAGGCAGGGC TTTCCGTGAA CGAGGAGAAC CTGCACTACG GCACCGTGCC    540
   TCTGCACGTG GCCTCGGGGT ACGACAACAC GCAGGGCTGC CTCAAGCTCC TCCGGCAGGG    600
   AGGAGACCCC ACCGTCGTGT CAGCCGCCGG ACGCACACCG ATCTCGAACA TGCTCGTCAA    660
   ACGCAACCAC GTGGCGGTCG CCGGCGCGCT GTCGACGCAC CCGAGCGCGG CAGTGGTCGT    720
   GCAGGCTCTC GAGCAGGCTC TCGAGAACGT GCTGAACGCC GGGCCCAGCG AGGCCTCGCG    780
   GCTCGCCGTG GCCTTTGTGG TGGCGCGCGC CGGCGCATCC GCGCTACCGG AGGCCGTGCG    840
   CCGTCTTCAC GAGGGCTTCG TCGCCGACTG CGAGCGCGAA GTCGCGTTGC TTTCCCGCAG    900
   CATGCTCGGC ACACCGGCCG TGAGCGCGCT GGTCGTGCTG GTCAGCAAGG AGGTCTTTGG    960
   CACTGTTATC TCCTCGCGTG CGCTGCGCGT CGCGCGGGAG GTCCGCGTGT ACGCAAGGCC   1020
   GCTCCGCGAG GCGCTCATAA ATCTGCGCCA CAAATGCCGC TTAGTTTCCA GCCTTAAAAG   1080
   GCAAGTGGGA CCCTGCTCGC TGCCCGGCGA ACTGGTGGAG CGCGTGCTCG CGACCGTGCC   1140
   ACTGGCCGAC TTGCGCCGCT CGTGCAGCCG CCGCGCGCCC GAGTGACTGC CCATCCCGTT   1200
   GCTGCGCGAC TCGGGACTGC CCTCTGTTTT TCTTTCCCGT TTCTTCTTAT TAGGTAGTTG 1260
   TTGCCCACCT CCATGATCCT CGCACGCGCT GGCGGGCGAC CTCGCACGCC CGCGGCGGCC 1320
   GCGGCGGCCG CCGAGGACGG CAAGAACAGT GATCGCCGGA AGCGCAAGCG CAAGACGCCC 1380
   AACTGCGAAG ACGCCGACAA CTCCGACGAC GAGCTAGCGC AGACGCCGTG CGACCGCGAG 1440
   TGGCCGGACT GTCGCGCGAG CTCGATCACG AGCTCCGACT CGGTCTCTCT CGGCGACGAG 1500
   ATCTACTTGC GGTACGTAGC CTCGCAGGTG GACTTCGCGC AGACCTGGGC CCCGCCGGTG 1560
   CGGCTGCTGC GCTTCTTCGG GAACTTCTCG AAGGAAACGC TCAGCCGCAT GTCGCGGCGC 1620
   GGGTACGTGA ACCGCTCCTA CTTCCAGATG GCGCACGCGC GCTTCTCGCC CACCAACGAC 1680
   GACATGTACC ACATGGCCAC TGGCGGGTAC GGCATCGTGT TCCGCTTCGA CCGCTACGTG 1740
   GTCAAGTACG TCTTCGAGCA CCGCAACGGC ATGTCCGAGA TGGACGCCTC TACGGAGTAC 1800
   ACGGTGCCGC GGTTCCTGCG CAATAACCTC AAGGGCGACG AGCGCGAGTT CGTGGTCTGC 1860
   GCGCTGGCCA TGGGGCTGAA CTACCGGCTG GGCTTCCTGC ACTCGCTGTA CCGGCGCGTG 1920
   CTGCACACGC TGCTGCTGCT CATGCGCGTG GAGGAAGGCC AGCGGCCCTC GGTAGAGATG 1980
   GCCAAGAAGC CGCTGCTGCG CTGGTTCGAG GCGCGCAAGG ACAGCGAGTC CTTCGTGCGC 2040
   CTGGTCTCGT ACTTCTACCC CTCGGCCGTG CAGAGCAACG TGAACCTGAT CAACAACTTC 2100
   CACCACCTGG TGCACTTCTT TGAGCACGAG AAGCGCGCGC GGTACGTGTT CGACCGCGGG 2160
   GCCGTGATCG TGTTCCCTCT GGCGCGCGGG TCCGCGGACT CGATCTCGCC GGAGGCGGCG 2220
   GCAGCGCTGG GCTTCGCGCC GCACTCGGAG TTCCTCAAGT TCGTGTTCCT GCAGATCGCG 2280
   CTGCTGTACC TGAAGATATA CGAGCTCCCG GGCTGCACGA ACTTCCTGCA CGTGGACCTG 2340
   AAGCCCGACA ACGTGCTCAT CTTCGACAGC GCGCGCGCTC AGCGTGACTG CGGCCGGTGC 2400
   GACTTTTCGC TTCGAAGAGC CCGTGCGCGC GGCGCTGAAC GACTTCGACT TCGCGCGCGT 2460
   GGCCACCATC GAGAACCGCA AGATCGCGGG CAGCGTCCGC GTGCCGCAGA ACTGGTACTA 2520
   CGACTTCCAC TTCTTCGCGC ACACGCTGCT GCGCGCGTAC CCGCACATCG CCGCGGAGGA 2580
   CCCGGGCTTC CACGCGCTGC TCTCGGAGCT CACGGTCTCG TGCTCGCGCG GGACCTGCGA 2640
   CCGCTTCCGG CTGCGCGTGT CCTCGCCGCA CCCCATCGAG CACCTCGCGC GGCTGGTGCG 2700
   CCGCGACGTC TTCTCCCGCT GGATAAATGC CGCCGCGGAC GCCCCCGACG CCGCACTCTC 2760
   CTGAGCCCAC GCCCGCGGCG CCGGGCTCGC TGTACGACGT CTTCCTCGCG CGCTTCCTGC 2820
   GCCAGCTGGC CGCGCGCGCG GCGCCGGCCT CGGCCGCCTG CGCCGTGCGC GTGGGTGCGG 2880
   TGCGCGGCCG CCTGCGGAAC TGCGAGCTGG TGGTGCTGAA CCGCTGCCAC GCGGACGCTG 2940
   CCGGCGCGCT CGCGCTGGCC TCCGCGGCGC TGGCGGAAAC GCTGGCGGAG CTGCCGCGCG 3000
   CGGACAGGCT CGCCGTCGCG CGCGAGCTGG GCGTGGACCC AGAGCACCCG GAGCTGACGC 3060
   CGGACCCCGC CTGCGCGGGC GAGAGGCGCG CTTGCGCAGA ACATCGACAT CCAGACGCTG 3120
   GACCTGGGCG ACTGCGGCGA CCCCAAAGGC CGCCGACTGC GCGTGGCGCT GGTGAACAGC 3180
   GGCCACGCGG CCGCAAACTG CGCGCTCGCG CGCGTAGCGA CCGCGCTGAC GCGCCGCGTG 3240
   CCCGCAAGCC GGCACGGCCT CGCGGAGGGC GGCACGCCGC CGTGGACGCT GCTGCTGGCG 3300
   GTGGCCGCGG TGACGGTGCT CAGCGTGGTG GCGGTTTCGC TGCTGCGGCG CGCGCTGCGG 3360
   GTGCGCTACC AATTCGCGCG GCCGGCCGCG CTGCGCGCGT AGCCGCGCAA AATGTAAATT 3420
   ATAACGCCCA ACTTTTAAGG GTGAGGCGCC ATGAAGTTTC TCGTCGGCAT ACTGGTAGCT 3480
   GTGTGCTTGC ACCAGTATCT GCTGAACGCG GACAGCACGA AAACATGGTC CGAAGTGTTT 3540
   GAAAACAGCG GGTGCAAGCC AAGGCCGATG GTCTTTCGAG TACACGACGA GCACCCGGAG 3600
   CTAACTTCTC AGCGGTTCAA CCCGCCGTGT GTCACGTTGA TGCGATGCGG CGGGTGCTGC 3660
   AACGACGAGA GCTTAGAATG CGTCCCCACG GAAGAGGCAA ACGTAACGAT GCAACTCATG 3720
   GGAGCGTCGG TCTCCGGTGG TAACGGGATG CAACATCTGA GCTTCGTAGA GCATAAGAAA 3780
   TGCGATTGTA AACCACCACT CACGACCACG CCACCGACGA CCACAAGGCC GCCCAGAAGA 3840
   CGCCGCTAGA ACTTTTTATG GACCGCATAT CCAAACGATG ATGCGATCAG GTCATGCGGA 3900
   AGGAGGCTCC ACGGAGCAAA GTGAAAAAGG ACCGCCTAGA GTCGAGACCC CTCCCTCCCG 3960
   CCTCGGGCAA ACCCACAGCC GCCGCAAACA CCACACCCGC CGACCTACCA TGCACCCCTC 4020
   GCCGCGCCGG CTGCTCGGCG CGCTCGCGCT GCTGGCGCTG GGCTTCGCTC GGCGCGCTCT 4080
   TCGCCCCGCG GCGCCGCTCG TGCCGGCCGC CTTCCTGGAG GTGGGGCACG TGCGCGCGAA 4140
   CCCGTCCGCC TCGGTGACCT GCCTCACGGT GGGCGGCGAC GGGCGGCACA TGGCGGCGGT 4200
   CGCGCACGGC GGCGGGACGC TCTCGCCGGT GTACCCGCTG GCCGCCGGCA TGCACGCGAC 4260
   CTTCTCCTCC GCGCGCAAGG GCGCGCTGCT GCTGAACGTC GCGACCGTGA CTGTGTACGA 4320
   CGTGCGCGCG CTCGCCCCCG AGTTCGAGCT CGTCTGCATC GCGGTGGTCG GCGGCTACAA 4380
   CTCGGCCGCG GCCGCCACGC GGCCCGCGGC CGAGTGGCAC CGCCAGCTGG AGCTGCGCCG 4440
   CTCGGAGCTG TGACCCCTCC CTCCCCGGTC TCCCTCTGTC TTTGTAATCG GCCTTAGAGA 4500
   TTAGACATCA TCCTCCACGC CTCTTTGTCC GCCGCCCTTC TTCGCGGACG GATGAACCAA 4560
   TTAATTAATT ATTTTTGTCG CTCGCCCGCT CACTCCGGCA AGGGAACGAG TGACGTTAAC    4620
   TCTCTCACCC TCACGCACAA GAACAAGAAC CGCTCACTCA CCGGGCAAGG GAACACGGTT    4680
   AAGGTCAACT CACTCGCGAG AACAAGTTGA CCCTCACTCT AGAGAACGAG GAACGGGCAA    4740
   CAAGCAACCG TCAACTCACT TACCACGAGA ACAAGTTGAC CGCCACTCAA AGGGAACAGA    4800
   GAACAGTAAC CGTTCTCGCT CGCTCGGAAC AATAGAACAA GTTAACGTCA ACTCGCTCGC    4860
   TCGGTGTAAG AGAACAACAG AACAAGCAAC TGTTGACCAC TCAACCCCCG GAGAAGAGAA    4920
   CAAGAGAGCA GTCAACTCAC CCACTCAGTC TTGGATGAGA GGAGGACGAG TTAACGAGTA    4980
   CTCGCACGCA GAGTGAGAGA GTGAGGACAT AATAATAGTT AACGAGTTAA TACTCACTCG    5040
   CTCACTCAGA GTGAGAGAGA ACCAGTGAGC GAGTTAACCG CGCACACGAG CGAGAGAACA    5100
   GTGAACTGCT CGCGCGCTCG CTCGGTAGCA GTCGGCCTTT CTTAAAACGG TTCGTAAAAC    5160
   TTTTCCCGAG ACAGTTCACC CTCCAAAACT TTTAAAACTA AACTCGGAGG TGGCCTGCCC    5220
   TCCACTCTCC GTAAAACTTT TGTAAAACTG TCGGAGGTCG GTCGACTTCG CAACTCGTCC    5280
   GCGAAAACTT TTCGTGGGCA GTGTCTGCCT CTCTCAGGCT CCTCGCATCA CTTTCGCGGA    5340
   GCCTCGAGGT AGGTCACCTC TCTCCAAACT TTTGTAAAAA CTTTTTCGCG GAGCCTCTGG    5400
   AGGCCGTCCT CCCTCCAAAA CTTTTCGTAA AATCTCTTCG GAGGCCGTCC TCCCTCCAAA    5460
   ACTTTTCGTA AAATCTTTGG GAGGTCGACC TCCCTCAAAA CTTTTTATAA AGCTT         5515
(2) INFORMATION FOR SEQ ID NO: 9:
   (i) SEQUENCE CHARACTERISTICS:
      (A) LENGTH: 1620 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Parapox ovis
      (B) STRAIN: D1701 Proteinkinase-Gen F10L (Version2)
   (xi) SEQUENCE DESCRIPTION: SEQ ID NO: 9:
   CGAGTGACTG CCCATCCCGT TGCTGCGCGA CTCGGGACTG CCCTCTGTTT TTCTTTCCCG    60
   TTTCTTCTTA TTAGGTAGTT GTTGCCCACC TCCATGATCC TCGCACGCGC TGGCGGGCGA 120
   CCTCGCACGC CCGCGGCGGC CGCGGCGGCC GCCGAGGACG GCAAGAACAG TGATCGCCGG 180
   AAGCGCAAGC GCAAGACGCC CAACTGCGAA GACGCCGACA ACTCCGACGA CGAGCTAGCG 240
   CAGACGCCGT GCGACCGCGA GTGGCCGGAC TGTCGCGCGA GCTCGATCAC GAGCTCCGAC 300
   TCGGTCTCTC TCGGCGACGA GATCTACTTG CGGTACGTAG CCTCGCAGGT GGACTTCGCG 360
   CAGACCTGGG CCCCGCCGGT GCGGCTGCTG CGCTTCTTCG GGAACTTCTC GAAGGAAACG 420
   CTCAGCCGCA TGTCGCGGCG CGGGTACGTG AACCGCTCCT ACTTCCAGAT GGCGCACGCG 480
   CGCTTCTCGC CCACCAACGA CGACATGTAC CACATGGCCA CTGGCGGGTA CGGCATCGTG 540
   TTCCGCTTCG ACCGCTACGT GGTCAAGTAC GTCTTCGAGC ACCGCAACGG CATGTCCGAG 600
   ATGGACGCCT CTACGGAGTA CACGGTGCCG CGGTTCCTGC GCAATAACCT CAAGGGCGAC 660
   GAGCGCGAGT TCGTGGTCTG CGCGCTGGCC ATGGGGCTGA ACTACCGGCT GGGCTTCCTG 720
   CACTCGCTGT ACCGGCGCGT GCTGCACACG CTGCTGCTGC TCATGCGCGT GGAGGAAGGC 780
   CAGCGGCCCT CGGTAGAGAT GGCCAAGAAG CCGCTGCTGC GCTGGTTCGA GGCGCGCAAG 840
   GACAGCGAGT CCTTCGTGCG CCTGGTCTCG TACTTCTACC CCTCGGCCGT GCAGAGCAAC 900
   GTGAACCTGA TCAACAACTT CCACCACCTG GTGCACTTCT TTGAGCACGA GAAGCGCGCG 960
   CGGTACGTGT TCGACCGCGG GGCCGTGATC GTGTTCCCTC TGGCGCGCGG GTCCGCGGAC 1020
   TCGATCTCGC CGGAGGCGGC GGCAGCGCTG GGCTTCGCGC GGCACTCGGA GTTCCTCAAG 1080
   TTCGTGTTCC TGCAGATCGC GCTGCTGTAC CTGAAGATAT ACGAGCTCCC GGGCTGCACG 1140
   AACTTCCTGC ACGTGGACCT GAAGCCCGAC AACGTGCTCA TCTTCGACAG CGCGCGCGCG 1200
   CTCAGCGTGA CTGCGGCCGG TGCGACTTTT CGCTTCGAAG AGCCCGTGCG CGCGGCGCTG 1260
   AACGACTTCG ACTTCGCGCG CGTGGCCACC ATCGAGAACC GCAAGATCGC GGGCAGCGTC 1320
   CGCGTGCCGC AGAACTGGTA CTACGACTTC CACTTCTTCG CGCACACGCT GCTGCGCGCG 1380
   TACCCGCACA TCGCCGCGGA GGACCCGGGC TTCCACGCGC TGCTCTCGGA GCTCACGGTC 1440
   TCGTGCTCGC GCGGGACCTG CGACCGCTTC CGGCTGCGCG TGTCCTCGCC GCACCCCATC 1500
   GAGCACCTCG CGCGGCTGGT GCGCCGCGAC GTCTTCTCCC GCTGGATAAA TGCCGCCGCG 1560
   GACGCCCCCG ACGCCGCACT CTCCTGAGCC CACGCCCGCG GCGCCGGGCT CGCTGTACGA 1620
(2) INFORMATION FOR SEQ ID NO: 10:
   (i) SEQUENCE CHARACTERISTICS:
      (A) LENGTH: 780 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Parapox ovis
      (B) STRAIN: D1701-F9L Gen, Version 2
   (xi) SEQUENCE DESCRIPTION: SEQ ID NO: 10:
   GAGCACCTCG CGCGGCTGGT GCGCCGCGAC GTCTTCTCCC GCTGGATAAA TGCCGCCGCG     60
   GACGCCCCCG ACGCCGCACT CTCCTGAGCC CACGCCCGCG GCGCCGGGCT CGCTGTACGA    120
   CGTCTTCCTC GCGCGCTTCC TGCGCCAGCT GGCCGCGCGC GCGGCGCCGG CCTCGGCCGC    180
   CTGCGCCGTG CGCGTGGGTG CGGTGCGCGG CCGCCTGCGG AACTGCGAGC TGGTGGTGCT    240
   GAACCGCTGC CACGCGGACG CTGCCGGCGC GCTCGCGCTG GCCTCCGCGG CGCTGGCGGA    300
   AACGCTGGCG GAGCTGCCGC GCGCGGACAG GCTCGCCGTC GCGCGCGAGC TGGGCGTGGA    360
   CCCAGAGCAC CCGGAGCTGA CGCCGGACCC CGCCTGCGCG GGCGAGAGCG CGCTTGCGCA    420
   GAACATCGAC ATCCAGACGC TGGACCTGGG CGACTGCGGC GACCCCAAAG GCCGCCGACT    480
   GCGCGTGGCG CTGGTGAACA GCGGCCACGC GGCCGCAAAC TGCGCGCTCG CGCGCGTAGC    540
   GACCGCGCTG ACGCGCCGCG TGCCCGCAAG CCGGCACGGC CTCGCGGAGG GCGGCACGCC    600
   GCCGTGGACG CTGCTGCTGG CGGTGGCCGC GGTGACGGTG CTCAGCGTGG TGGCGGTTTC    660
   GCTGCTGCGG CGCGCGCTGC GGGTGCGCTA CCAATTCGCG CGGCCGGCCG CGCTGCGCGC    720
   GTAGCCGCGC AAAATGTAAA TTATAACGCC CAACTTTTAA GGGTGAGGCG CCATGAAGTT    780
(2) INFORMATION FOR SEQ ID NO: 11:
   (i) SEQUENCE CHARACTERISTICS:
      (A) LENGTH: 297 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Parapox ovis
      (B) STRAIN: D1701 10kD- Gen
   (xi) SEQUENCE DESCRIPTION: SEQ ID NO: 11:
   CAATATGGAG GAAAATGACG GAGAAAACCT ATTGGCTCAG CCTGATGATG ATACAGACAA    60
   TTTAACCAAC GGAGTGTACG CGGCTGGAGC TCCAACTAAA GAAAGTGTGG AAGAGCGTCT   120
   CGTAAGCTTG TTAGACGGTT ACAAAAATAT AACTGATTGC TGCAGAGAAA CAGGTAACCG   180
   GTTAGACAGA CTAGAAAGAC ACTTGGAGAG TCTACGTAAA GCTCTTCTTG ATCTCAACAG   240
   AAAAATAGAT GTACAGACAG GATACAGCAG ATATTAGATA CCGCTGTGTT GCGTCTG      297
(2) INFORMATION FOR SEQ ID NO: 12:
   (i) SEQUENCE CHARACTERISTICS:
      (A) LENGTH: 5519 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Parapox ovis
      (B) STRAIN: D 1701, HindIII-Fragment I, Version 2
   (xi) SEQUENCE DESCRIPTION: SEQ ID NO: 12:
   AAGCTTGTTG CGCGAGTACG TGGTGACCCG CGCCTACTCG GATCAGACCG AGCCGATCAT    60
   GGACTTGCTC ATCGGCATGG GCGCCGACGT GGACATGCAG GTCGGCGTGT GCCGCACGGC   120
   GCTGCACGCC TGCCTTACGG GCTTGAACAC GAACCCGTGC ATGATTCGCG CGCTGCTTCG   180
   GCGCGGCGCC AGCGTGACCG CAAAAGACAC CTACGAGATG ACGCCACTGG CGGTGTTGCT   240
   GAAGTCCGCG AGCGCGACGC CGGAGCTCGT GCGCATCCTC GTGGAAGCAG GCTCCGACGT   300
   GAGCGCCACC GACTTCCGCC TCAACGGCAT GCTGCACCAG CACGCGCAGT CCACGCGCCC   360
   GCGCGCGAGC GTCATGCGCG AGCTCATCCG GCTGGGGTGC AGCCCAGCGG CCAAAAACAT   420
   GTTTGGGAAC ACGCCGATGC ACATGCTGGC CATGGAAAGC TCCTGCCGCC GCTCGCTGAT   480
   CCTCCCGCTG CTGGAGGCAG GGCTTTCCGT GAACGAGGAG AACCTGCACT ACGGCACCGT   540
   GCCTCTGCAC GTGGCCTCGG GGTACGACAA CACGCAGGGC TGCCTCAAGC TCCTCCGGCA   600
   GGGAGGAGAC CCCACCGTCG TGTCAGCCGC CGGACGCACA CCGATCTCGA ACATGCTCGT   660
   CAAACGCAAC CACGTGGCGG TCGCCGGCGC GCTGTCGACG CACCCGAGCG CGGCAGTGGT   720
   CGTGCAGGCT CTCGAGCAGG CTCTCGAGAA CGTGCTGAAC GCCGGGCCCA GCGAGGCCTC   780
   GCGGCTCGCC GTGGCCTTTG TGGTGGCGCG CGCCGGCGCA TCCGCGCTAC CGGAGGCCGT   840
   GCGCCGTCTT CACGAGGGCT TCGTCGCCGA CTGCGAGCGC GAAGTCGCGT TGCTTTCCCG   900
   CAGCATGCTC GGCACACCGG CCGTGAGCGC GCTGGTCGTG CTGGTCAGCA AGGAGGTCTT   960
   TGGCACTGTT ATCTCCTCGC GTGCGCTGCG CGTCGCGCGG GAGGTCCGCG TGTACGCAAG  1020
   GCCGCTCCGC GAGGCGCTCA TAAATCTGCG CCACAAATGC CGCTTAGTTT CCAGCCTTAA  1080
   AAGGCAAGTG GGACCCTGCT CGCTGCCCGG CGAACTGGTG GAGCGCGTGC TCGCGACCGT  1140
   GCCACTGGCC GACTTGCGCC GCTCGTGCAG CCGCCGCGCG CCCGAGTGAC TGCCCATCCC  1200
   GTTGCTGCGC GACTCGGGAC TGCCCTCTGT TTTTCTTTCC CGTTTCTTCT TATTAGGTAG  1260
   TTGTTGCCCA CCTCCATGAT CCTCGCACGC GCTGGCGGGC GACCTCGCAC GCCCGCGGCG  1320
   GCCGCGGCGG CCGCCGAGGA CGGCAAGAAC AGTGATCGCC GGAAGCGCAA GCGCAAGACG  1380
   CCCAACTGCG AAGACGCCGA CAACTCCGAC GACGAGCTAG CGCAGACGCC GTGCGACCGC  1440
   GAGTGGCCGG ACTGTCGCGC GAGCTCGATC ACGAGCTCCG ACTCGGTCTC TCTCGGCGAC  1500
   GAGATCTACT TGCGGTACGT AGCCTCGCAG GTGGACTTCG CGCAGACCTG GGCCCCGCCG  1560
   GTGCGGCTGC TGCGCTTCTT CGGGAACTTC TCGAAGGAAA CGCTCAGCCG CATGTCGCGG  1620
   CGCGGGTACG TGAACCGCTC CTACTTCCAG ATGGCGCACG CGCGCTTCTC GCCCACCAAC  1680
   GACGACATGT ACCACATGGC CACTGGCGGG TACGGCATCG TGTTCCGCTT CGACCGCTAC  1740
   GTGGTCAAGT ACGTCTTCGA GCACCGCAAC GGCATGTCCG AGATGGACGC CTCTACGGAG 1800
   TACACGGTGC CGCGGTTCCT GCGCAATAAC CTCAAGGGCG ACGAGCGCGA GTTCGTGGTC 1860
   TGCGCGCTGG CCATGGGGCT GAACTACCGG CTGGGCTTCC TGCACTCGCT GTACCGGCGC 1920
   GTGCTGCACA CGCTGCTGCT GCTCATGCGC GTGGAGGAAG GCCAGCGGCC CTCGGTAGAG 1980
   ATGGCCAAGA AGCCGCTGCT GCGCTGGTTC GAGGCGCGCA AGGACAGCGA GTCCTTCGTG 2040
   CGCCTGGTCT CGTACTTCTA CCCCTCGGCC GTGCAGAGCA ACGTGAACCT GATCAACAAC 2100
   TTCCACCACC TGGTGCACTT CTTTGAGCAC GAGAAGCGCG CGCGGTACGT GTTCGACCGC 2160
   GGGGCCGTGA TCGTGTTCCC TCTGGCGCGC GGGTCCGCGG ACTCGATCTC GCCGGAGGCG 2220
   GCGGCAGCGC TGGGCTTCGC GCCGCACTCG GAGTTCCTCA AGTTCGTGTT CCTGCAGATC 2280
   GCGCTGCTGT ACCTGAAGAT ATACGAGCTC CCGGGCTGCA CGAACTTCCT GCACGTGGAC 2340
   CTGAAGCCCG ACAACGTGCT CATCTTCGAC AGCGCGCGCG CGCTCAGCGT GACTGCGGCC 2400
   GGTGCGACTT TTCGCTTCGA AGAGCCCGTG CGCGCGGCGC TGAACGACTT CGACTTCGCG 2460
   CGCGTGGCCA CCATCGAGAA CCGCAAGATC GCGGGCAGCG TCCGCGTGCC GCAGAACTGG 2520
   TACTACGACT TCCACTTCTT CGCGCACACG CTGCTGCGCG CGTACCCGCA CATCGCCGCG 2580
   GAGGACCCGG GCTTCCACGC GCTGCTCTCG GAGCTCACGG TCTCGTGCTC GCGCGGGACC 2640
   TGCGACCGCT TCCGGCTGCG CGTGTCCTCG CCGCACCCCA TCGAGCACCT CGCGCGGCTG 2700
   GTGCGCCGCG ACGTCTTCTC CCGCTGGATA AATGCCGCCG CGGACGCCCC CGACGCCGCA 2760
   CTCTCCTGAG CCCACGCCCG CGGCGCCGGG CTCGCTGTAC GACGTCTTCC TCGCGCGCTT 2820
   CCTGCGCCAG CTGGCCGCGC GCGCGGCGCC GGCCTCGGCC GCCTGCGCCG TGCGCGTGGG 2880
   TGCGGTGCGC GGCCGCCTGC GGAACTGCGA GCTGGTGGTG CTGAACCGCT GCCACGCGGA 2940
   CGCTGCCGGC GCGCTCGCGC TGGCCTCCGC GGCGCTGGCG GAAACGCTGG CGGAGCTGCC 3000
   GCGCGCGGAC AGGCTCGCCG TCGCGCGCGA GCTGGGCGTG GACCCAGAGC ACCCGGAGCT 3060
   GACGCCGGAC CCCGCCTGCG CGGGCGAGAG CGCGCTTGCG CAGAACATCG ACATCCAGAC 3120
   GCTGGACCTG GGCGACTGCG GCGACCCCAA AGGCCGCCGA CTGCGCGTGG CGCTGGTGAA 3180
   CAGCGGCCAC GCGGCCGCAA ACTGCGCGCT CGCGCGCGTA GCGACCGCGC TGACGCGCCG 3240
   CGTGCCCGCA AGCCGGCACG GCCTCGCGGA GGGCGGCACG CCGCCGTGGA CGCTGCTGCT 3300
   GGCGGTGGCC GCGGTGACGG TGCTCAGCGT GGTGGCGGTT TCGCTGCTGC GGCGCGCGCT 3360
   GCGGGTGCGC TACCAATTCG CGCGGCCGGC CGCGCTGCGC GCGTAGCCGC GCAAAATGTA 3420
   AATTATAACG CCCAACTTTT AAGGGTGAGG CGCCATGAAG TTTCTCGTCG GCATACTGGT 3480
   AGCTGTGTGC TTGCACCAGT ATCTGCTGAA CGCGGACAGC ACGAAAACAT GGTCCGAAGT 3540
   GTTTGAAAAC AGCGGGTGCA AGCCAAGGCC GATGGTCTTT CGAGTACACG ACGAGCACCC 3600
   GGAGCTAACT TCTCAGCGGT TCAACCCGCC GTGTGTCACG TTGATGCGAT GCGGCGGGTG 3660
   CTGCAACGAC GAGAGCTTAG AATGCGTCCC CACGGAAGAG GCAAACGTAA CGATGCAACT 3720
   CATGGGAGCG TCGGTCTCCG GTGGTAACGG GATGCAACAT CTGAGCTTCG TAGAGCATAA 3780
   GAAATGCGAT TGTAAACCAC CACTCACGAC CACGCCACCG ACGACCACAA GGCCGCCCAG 3840
   AAGACGCCGC TAGAACTTTT TATGGACCGC ATATCCAAAC GATGATGCGA TCAGGTCATG 3900
   CGGAAGGAGG CTCCACGGAG CAAAGTGAAA AAGGACCGCC TAGAGTCGAG ACCCCTCCCT 3960
   CCCGCCTCGG GCAAACCCAC AGCCGCCGCA AACACCACAC CCGCCGACCT ACCATGCACC 4020
   CCTCGCCGCG CCGGCTGCTC GGCGCGCTCG CGCTGCTGGC GCTGGGCTTC GCTCGGCGCG 4080
   CTCTTCGCCC CGCGGCGCCG CTCGTGCCGG CCGCCTTCCT GGAGGTGGGG CACGTGCGCG 4140
   CGAACCCGTC CGCCTCGGTG ACCTGCCTCA CGGTGGGCGG CGACGGGCGG CACATGGCGG 4200
   CGGTCGCGCA CGGCGGCGGG ACGCTCTCGC CGGTGTACCC GCTGGCCGCC GGCATGCACG 4260
   CGACCTTCTC CTCCGCGCGC AAGGGCGCGC TGCTGCTGAA CGTCGCGACC GTGACTGTGT 4320
   ACGACGTGCG CGCGCTCGCC CCCGAGTTCG AGCTCGTCTG CATCGCGGTG GTCGGCGGCT 4380
   ACAACTCGGC CGCGGCCGCC ACGCGGCCCG CGGCCGAGTG GCACCGCCAG CTGGAGCTGC 4440
   GCCGCTCGGA GCTGTGACCC CTCCCTCCCC GGTCTCCCTC TGTCTTTGTA ATCGGCCTTA 4500
   GAGATTAGAC ATCATCCTCC ACGCCTCTTT GTCCGCCGCC CTTCTTCGCG GACGGATGAA 4560
   CCAATTAATT AATTATTTTT GTCGCTCGCC CGCTCACTCC GGCAAGGGAA CGAGTGACGT 4620
   TAACTCTCTC ACCCTCACGC ACAAGAACAA GAACCGCTCA CTCACCGGGC AAGGGAACAC 4680
   GGTTAAGGTC AACTCACTCG CGAGAACAAG TTGACCCTCA CTCTAGAGAA CGAGGAACGG 4740
   GCAACAAGCA ACCGTCAACT CACTTACCAC GAGAACAAGT TGACCGCCAC TCAAAGGGAA 4800
   CAGAGAACAG TAACCGTTCT CGCTCGCTCG GAACAATAGA ACAAGTTAAC GTCAACTCGC 4860
   TCGCTCGGTG TAAGAGAACA ACAGAACAAG CAACTGTTGA CCACTCAACC CCCGGAGAAG 4920
   AGAACAAGAG AGCAGTCAAC TCACCCACTC AGTCTTGGAT GAGAGGAGGA CGAGTTAACG 4980
   AGTACTCGCA CGCAGAGTGA GAGAGTGAGG ACATAATAAT AGTTAACGAG TTAATACTCA 5040
   CTCGCTCACT CAGAGTGAGA GAGAACCAGT GAGCGAGTTA ACCGCGCACA CGAGCGAGAG 5100
   AACAGTGAAC TGCTCGCGCG CTCGCTCGGT AGCAGTCGGC CTTTCTTAAA ACGGTTCGTA 5160
   AAACTTTTCC CGAGACAGTT CACCCTCCAA AACTTTTAAA ACTAAACTCG GAGGTGGCCT 5220
   GCCCTCCACT CTCCGTAAAA CTTTTGTAAA ACTGTCGGAG GTCGGTCGAC TTCGCAACTC 5280
   GTCCGCGAAA ACTTTTCGTG GGCAGTGTCT GCCTCTCTCA GGCTCCTCGC ATCACTTTCG 5340
   CGGAGCCTCG AGGTAGGTCA CCTCTCTCCA AACTTTTGTA AAAACTTTTT CGCGGAGCCT 5400
   CTGGAGGCCG TCCTCCCTCC AAAACTTTTC GTAAAATCTC TTCGGAGGCC GTCCTCCCTC 5460
   CAAAACTTTT CGTAAAATCT TTGGGAGGTC GACCTCCCTC AAAACTTTTT ATAAAGCTT 5519
(2) INFORMATION FOR SEQ ID NO: 13:
   (i) SEQUENCE CHARACTERISTICS:
      (A) LENGTH: 1742 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Parapox ovis
      (B) STRAIN: D 1701 Proteinkinase-Gen F10L (Version 3)
   (xi) SEQUENCE DESCRIPTION: SEQ ID NO: 13:
   CGAGTGACTG CCCATCCCGT TGCTGCGCGA CTCGGGACTG CCCTCTGTTT TTCTTTCCCG 60
   TTTCTTCTTA TTAGGTAGTT GTTGCCCACC TCCATGATCC TCGCACGCGC TGGCGGGCGA 120
   CCTCGCACGC CCGCGGCGGC CGCGGCGGCC GCCGAGGACG GCAAGAACAG TGATCGCCGG 180
   AAGCGCAAGC GCAAGACGCC CAACTGCGAA GACGCCGACA ACTCCGACGA CGAGCTAGCG 240
   CAGACGCCGT GCGACCGCGA GTGGCCGGAC TGTCGCGCGA GCTCGATCAC GAGCTCCGAC 300
   TCGGTCTCTC TCGGCGACGA GATCTACTTG CGGTACGTAG CCTCGCAGGT GGACTTCGCG 360
   CAGACCTGGG CCCCGCCGGT GCGGCTGCTG CGCTTCTTCG GGAACTTCTC GAAGGAAACG 420
   CTCAGCCGCA TGTCGCGGCG CGGGTACGTG AACCGCTCCT ACTTCCAGAT GGCGCACGCG 480
   CGCTTCTCGC CCACCAACGA CGACATGTAC CACATGGCCA CTGGCGGGTA CGGCATCGTG 540
   TTCCGCTTCG ACCGCTACGT GGTCAAGTAC GTCTTCGAGC ACCGCAACGG CATGTCCGAG 600
   ATGGACGCCT CTACGGAGTA CACGGTGCCG CGGTTCCTGC GCAATAACCT CAAGGGCGAC    660
   GAGCGCGAGT TCGTGGTCTG CGCGCTGGCC ATGGGGCTGA ACTACCGGCT GGGCTTCCTG    720
   CACTCGCTGT ACCGGCGCGT GCTGCACACG CTGCTGCTGC TCATGCGCGT GGAGGAAGGC    780
   CAGCGGCCCT CGGTAGAGAT GGCCAAGAAG CCGCTGCTGC GCTGGTTCGA GGCGCGCAAG    840
   GACAGCGAGT CCTTCGTGCG CCTGGTCTCG TACTTCTACC CCTCGGCCGT GCAGAGCAAC    900
   GTGAACCTGA TCAACAACTT CCACCACCTG GTGCACTTCT TTGAGCACGA GAAGCGCGCG    960
   CGGTACGTGT TCGACCGCGG GGCCGTGATC GTGTTCCCTC TGGCGCGCGG GTCCGCGGAC   1020
   TCGATCTCGC CGGAGGCGGC GGCAGCGCTG GGCTTCGCGC CGCACTCGGA GTTCCTCAAG   1080
   TTCGTGTTCC TGCAGATCGC GCTGCTGTAC CTGAAGATAT ACGAGCTCCC GGGCTGCACG   1140
   AACTTCCTGC ACGTGGACCT GAAGCCCGAC AACGTGCTCA TCTTCGACAG CGCGCGCGCG   1200
   CTCAGCGTGA CTGCGGCCGG TGCGACTTTT CGCTTCGAAG AGCCCGTGCG CGCGGCGCTG   1260
   AACGACTTCG ACTTCGCGCG CGTGGCCACC ATCGAGAACC GCAAGATCGC GGGCAGCGTC   1320
   CGCGTGCCGC AGAACTGGTA CTACGACTTC CACTTCTTCG CGCACACGCT GCTGCGCGCG   1380
   TACCCGCACA TCGCCGCGGA GGACCCGGGC TTCCACGCGC TGCTCTCGGA GCTCACGGTC   1440
   TCGTGCTCGC GCGGGACCTG CGACCGCTTC CGGCTGCGCG TGTCCTCGCC GCACCCCATC   1500
   GAGCACCTCG CGCGGCTGGT GCGCCGCGAC GTCTTCTCCC GCTGGATAAA TGCCGCCGCG   1560
   GACGCCCCCG ACGCCGCACT CTCCTGAGCC CACGCCCGCG GCGCCGGGCT CGCTGTACGA   1620
   CGTCTTCCTC GCGCGCTTCC TGCGCCAGCT GGCCGCGCGC GCGGCGCCGG CCTCGGCCGC   1680
   CTGCGCCGTG CGCGTGGGTG CGGTGCGCGG CCGCCTGCGG AACTGCGAGC TGGTGGTGCT   1740
   GA                                                                  1742
(2) INFORMATION FOR SEQ ID NO: 14:
   (i) SEQUENCE CHARACTERISTICS:
      (A) LENGTH: 497 amino acids
      (B) TYPE: amino acid
      (C) STRANDEDNESS: single
      (D) TOPOLOGY: linear
   (ii) MOLECULE TYPE: protein
   (iii) HYPOTHETICAL: NO
   (iv) ANTI-SENSE: NO
   (vi) ORIGINAL SOURCE:
      (A) ORGANISM: Parapox ovis
      (B) STRAIN: D1701 Proteinkinase F10L
   (xi) SEQUENCE DESCRIPTION: SEQ ID NO: 14:
   Met Ile Leu Ala Arg Ala Gly Gly Arg Pro Arg Thr Pro Ala Ala Ala
   1               5                   10                  15
   Ala Ala Ala Ala Glu Asp Gly Lys Asn Ser Asp Arg Arg Lys Arg Lys
               20                  25                  30
   Arg Lys Thr Pro Asn Cys Glu Asp Ala Asp Asn Ser Asp Asp Glu Leu
           35                  40                  45
   Ala Gln Thr Pro Cys Asp Arg Glu Trp Pro Asp Cys Arg Ala Ser Ser
       50                  55                  60
   Ile Thr Ser Ser Asp Ser Val Ser Leu Gly Asp Glu Ile Tyr Leu Arg
   65                  70                  75                  80
   Tyr Val Ala Ser Gln Val Asp Phe Ala Gln Thr Trp Ala Pro Pro Val
                   85                  90                  95
   Arg Leu Leu Arg Phe Phe Gly Asn Phe Ser Lys Glu Thr Leu Ser Arg
               100                 105                 110
   Met Ser Arg Arg Gly Tyr Val Asn Arg Ser Tyr Phe Gln Met Ala His
           115                 120                 125
   Ala Arg Phe Ser Pro Thr Asn Asp Asp Met Tyr His Met Ala Thr Gly
       130                 135                 140
   Gly Tyr Gly Ile Val Phe Arg Phe Asp Arg Tyr Val Val Lys Tyr Val
   145                 150                 155                 160
   Phe Glu His Arg Asn Gly Met Ser Glu Met Asp Ala Ser Thr Glu Tyr
                   165                 170                 175
   Thr Val Pro Arg Phe Leu Arg Asn Asn Leu Lys Gly Asp Glu Arg Glu
               180                 185                 190
   Phe Val Val Cys Ala Leu Ala Met Gly Leu Asn Tyr Arg Leu Gly Phe
           195                 200                 205
   Leu His Ser Leu Tyr Arg Arg Val Leu His Thr Leu Leu Leu Leu Met
       210                 215                 220
   Arg Val Glu Glu Gly Gln Arg Pro Ser Val Glu Met Ala Lys Lys Pro
   225                 230                 235                 240
   Leu Leu Arg Trp Phe Glu Ala Arg Lys Asp Ser Glu Ser Phe Val Arg
                   245                 250                 255
   Leu Val Ser Tyr Phe Tyr Pro Ser Ala Val Gln Ser Asn Val Asn Leu
               260                 265                 270
   Ile Asn Asn Phe His His Leu Val His Phe Phe Glu His Glu Lys Arg
           275                 280                 285
   Ala Arg Tyr Val Phe Asp Arg Gly Ala Val Ile Val Phe Pro Leu Ala
       290                 295                 300
   Arg Gly Ser Ala Asp Ser Ile Ser Pro Glu Ala Ala Ala Ala Leu Gly
   305                 310                 315                 320
   Phe Ala Pro His Ser Glu Phe Leu Lys Phe Val Phe Leu Gln Ile Ala
                   325                 330                 335
   Leu Leu Tyr Leu Lys Ile Tyr Glu Leu Pro Gly Cys Thr Asn Phe Leu
               340                 345                 350
   His Val Asp Leu Lys Pro Asp Asn Val Leu Ile Phe Asp Ser Ala Arg
           355                 360                 365
   Ala Leu Ser Val Thr Ala Ala Gly Ala Thr Phe Arg Phe Glu Glu Pro
       370                 375                 380
   Val Arg Ala Ala Leu Asn Asp Phe Asp Phe Ala Arg Val Ala Thr Ile
   385                 390                 395                 400
   Glu Asn Arg Lys Ile Ala Gly Ser Val Arg Val Pro Gln Asn Trp Tyr
                   405                 410                 415
   Tyr Asp Phe His Phe Phe Ala His Thr Leu Leu Arg Ala Tyr Pro His
               420                 425                 430
   Ile Ala Ala Glu Asp Pro Gly Phe His Ala Leu Leu Ser Glu Leu Thr
           435                 440                 445
   Val Ser Cys Ser Arg Gly Thr Cys Asp Arg Phe Arg Leu Arg Val Ser
       450                 455                 460
   Ser Pro His Pro Ile Glu His Leu Ala Arg Leu Val Arg Arg Asp Val
   465                 470                 475                 480
   Phe Ser Arg Trp Ile Asn Ala Ala Ala Asp Ala Pro Asp Ala Ala Leu
                   485                 490                 495
   Ser
(2) INFORMATION FOR SEQ ID NO: 15:
   (i) SEQUENCE CHARACTERISTICS:
      (A) LENGTH: 132 amino acids
      (B) TYPE: amino acid
      (C) STRANDEDNESS: single
      (D) TOPOLOGY: linear
   (ii) MOLECULE TYPE: protein
   (iii) HYPOTHETICAL: NO
   (iv) ANTI-SENSE: NO
   (vi) ORIGINAL SOURCE:
      (A) ORGANISM: Parapox ovis
      (B) STRAIN: D1701 VEGF- Protein
   (xi) SEQUENCE DESCRIPTION: SEQ ID NO: 15:
   Met Lys Phe Leu Val Gly Ile Leu Val Ala Val Cys Leu His Gln Tyr
   1               5                   10                  15
   Leu Leu Asn Ala Asp Ser Thr Lys Thr Trp Ser Glu Val Phe Glu Asn
               20                  25                  30
   Ser Gly Cys Lys Pro Arg Pro Met Val Phe Arg Val His Asp Glu His
           35                  40                  45
   Pro Glu Leu Thr Ser Gln Arg Phe Asn Pro Pro Cys Val Thr Leu Met
       50                  55                  60
   Arg Cys Gly Gly Cys Cys Asn Asp Glu Ser Leu Glu Cys Val Pro Thr
   65                  70                  75                  80
   Glu Glu Ala Asn Val Thr Met Gln Leu Met Gly Ala Ser Val Ser Gly
                   85                  90                  95
   Gly Asn Gly Met Gln His Leu Ser Phe Val Glu His Lys Lys Cys Asp
               100                 105                 110
   Cys Lys Pro Pro Leu Thr Thr Thr Pro Pro Thr Thr Thr Arg Pro Pro
           115                 120                 125
   Arg Arg Arg Arg
       130
(2) INFORMATION FOR SEQ ID NO: 16:
   (i) SEQUENCE CHARACTERISTICS:
      (A) LENGTH: 224 amino acids
      (B) TYPE: amino acid
      (C) STRANDEDNESS: single
      (D) TOPOLOGY: linear
   (ii) MOLECULE TYPE: protein
   (iii) HYPOTHETICAL: NO
   (iv) ANTI-SENSE: NO
   (vi) ORIGINAL SOURCE:
      (A) ORGANISM: Parapox ovis
      (B) STRAIN: D1701 Protein F9L
   (xi) SEQUENCE DESCRIPTION: SEQ ID NO: 16:

### Verzeichnis der Abbildungen

- Abb. 1: zeigt die physikalische Karte des HindIII-Fragment I aus Orf D1701 in den Plasmiden pORF-1/-2. Die dünnen Pfeile markieren die identifizierten mRNAs, die dicken Pfeile ORFs.
- Abb. 2: zeigt die physikalische Karte der Hind III Erkennungsstellen auf dem D1701-Genom und die auf dem Hind III-Fragment I identifizierten Gene sowie einen Teil der Inverted Terminal Repeat Region.
- Abb. 3: zeigt Plasmid pCE4. Nach NruI-Spaltung wurde ein 396bp Fragment gegen die LacZ-Kassette ausgetauscht.

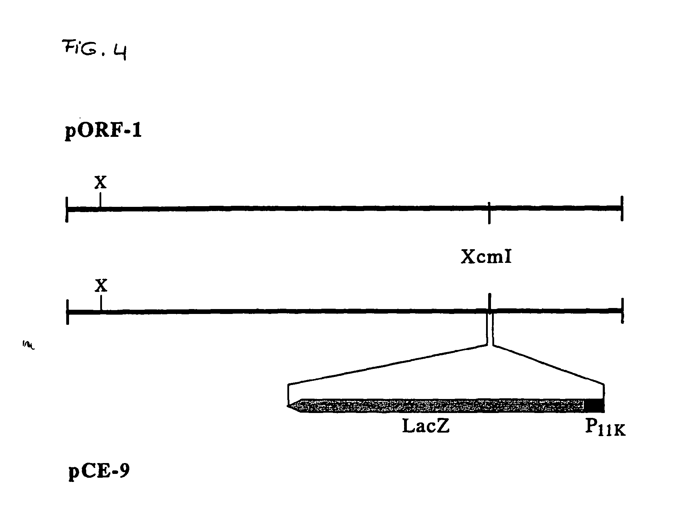

- Abb. 4: zeigt Plasmid pCE9 in das nach Linearsierung durch XcmI-Spaltung die LacZ-Kassette inseriert wurde.
- Abb. 5: zeigt schematisch, wie im Text beschrieben, die Strategie zur Erzeugung neuer singulärer Schnittstellen durch PCR.
- Abb. 6: zeigt schematisch die bidirektionale Verkürzung durch die Nuklease Bal31 mit anschließender Insertion der EcoRV-Linker und LacZ-Kassette.
- Abb. 7: zeigt die Strategie zur Herstellung der LacZ/gpt-Selektionskassette:
P_{11K}: Vaccinia 11k-Promotor
P_{VEGF}: PPV VEGF-Promotor
Sm: Sma I
B: Bam H1
- Abb. 8: zeigt schematisch die Klonierungsstrategie des EcoR1-Fragmentes E von PPV D1701, das das 10 kDA Gen enthält (wie im Text beschrieben).

## Patentansprüche

1. Rekombinant hergestellte PPV mit Insertionen und/oder Deletionen, enthaltend ein Fremdantigen, welches einen erregerspezifischen Schutz induzieren kann.

2. Rekombinant hergestellte PPV mit Insertionen und/oder Deletionen, enthaltend ein Zytokin.

3. Rekombinant hergestellte PPV nach Anspruch 1 oder 2, enthaltend Insertionen und/oder Deletionen in Genomabschnitten, die nicht für die Virusvermehrung notwending sind.

4. Rekombinant hergestellte PPV nach Anspruch 1 oder 2, enthaltend Insertionen und/oder Deletionen in Genomabschnitten, die für die Virusvermehrung notwending sind.

## Claims

1. Recombinantly prepared PPVs with insertions and/or deletions, containing a foreign antigen which can induce a pathogen-specific protection.

2. Recombinantly prepared PPVs with insertions and/or deletions, containing a cytokine.

3. Recombinantly prepared PPVs according to Claim 1 or 2, containing insertions and/or deletions in genome segments which are not required for multiplication of the virus.

4. Recombinantly prepared PPVs according to Claim 1 or 2, containing insertions and/or deletions in genome segments which are required for multiplication of the virus.

## Revendications

1. PPV produits par recombinaison avec insertions et/ou délétions, contenant un antigène étranger capable d'induire une protection qui est spécifique d'un agent pathogène.

2. PPV produits par recombinaison avec insertions et/ou délétions, contenant une cytokine.

3. PPV produits par recombinaison selon la revendication 1 ou 2, contenant des insertions et/ou présentant des délétions dans des parties du génome qui ne sont pas nécessaires à la multiplication du virus.

4. PPV produits par recombinaison selon la revendication 1 ou 2, contenant des insertions et/ou présentant des délétions dans des parties du génome qui sont nécessaires à la multiplication du virus.
